# EUROPEAN PATENT SPECIFICATION

(11) **EP 3 280 801 B1**
(45) Date of publication and mention of the grant of the patent: **25.11.2020**
(21) Application number: 16718219.5
(22) Date of filing: 06.04.2016
(51) Int. Cl.: C12N 9/64, A61K 38/17, A61P 25/28, A61P 35/00, A61P 43/00

(54) **INTERNALISATION OF HUMAN HTRA1 AND CARGO PROTEINS INTO MAMMALIAN CELLS**
INTERNALISIERUNG VON MENSCHLICHEN HTRA1- UND CARGOPROTEINEN IN SÄUGETIERZELLEN
INTERNALISATION DE PROTÉINES HTRA1 ET CARGO HUMAINES DANS DES CELLULES DE MAMMIFÈRE

(30) Priority: 07.04.2015 EP 15162558
(43) Date of publication of application: 14.02.2018
(73) Proprietor: Universität Duisburg-Essen, 45141 Essen (DE)
(72) Inventor: EHRMANN, Michael, 45138 Essen (DE); NELLES, Jasmin, 46045 Oberhausen (DE); POEPSEL, Simon, 58256 Ennepetal (DE); SEVERIN, Katharina, 45130 Essen (DE)
(74) Representative: Hemsath, Lars
(86) International application number: PCT/EP2016/057462
(87) International publication number: WO 2016/162346

(56) References cited:
- WO-A2-2008/013893
- ANDRÉ N. TIADEN ET AL: "Human Serine Protease HTRA1 Positively Regulates Osteogenesis of Human Bone Marrow-derived Mesenchymal Stem Cells and Mineralization of Differentiating Bone-forming Cells Through the Modulation of Extracellular Matrix Protein", STEM CELLS, vol. 30, no. 10, 20 October 2012 (2012-10-20), pages 2271-2282, XP055211450, ISSN: 1066-5099, DOI: 10.1002/stem.1190
- CHARLES EIGENBROT ET AL: "Structural and Functional Analysis of HtrA1 and Its Subdomains", STRUCTURE, CURRENT BIOLOGY LTD., PHILADELPHIA, PA, US, vol. 20, no. 6, 30 March 2012 (2012-03-30) , pages 1040-1050, XP028520147, ISSN: 0969-2126, DOI: 10.1016/J.STR.2012.03.021 [retrieved on 2012-04-13]
- JING L. GUO ET AL: "Seeding of Normal Tau by Pathological Tau Conformers Drives Pathogenesis of Alzheimer-like Tangles", JOURNAL OF BIOLOGICAL CHEMISTRY, vol. 286, no. 17, 3 March 2011 (2011-03-03), pages 15317-15331, XP055693541, US ISSN: 0021-9258, DOI: 10.1074/jbc.M110.209296
- TIM CLAUSEN ET AL: "HTRA proteases: regulated proteolysis in protein quality control", NATURE REVIEWS MOLECULAR CELL BIOLOGY, vol. 12, no. 3, 16 February 2011 (2011-02-16), pages 152-162, XP055187235, ISSN: 1471-0072, DOI: 10.1038/nrm3065
- Spillantini ET AL: "Tau pathology and neurodegeneration", Lancet Neurology, The, 1 January 2013 (2013-01-01), pages 609-622, XP055693543, England DOI: 10.1016/S1474-4422(13)70090-5 Retrieved from the Internet: URL:https://reader.elsevier.com/reader/sd/ pii/S1474442213700905?token=0042B7100945A2 C25B60FC91F6677F0E32ED9339758903EADA537309 090B8D16B231038A50301C44F27F284BFB748CBB [retrieved on 2020-05-11]
- ANNA LEHNER ET AL: "Downregulation of Serine Protease HTRA1 Is Associated with Poor Survival in Breast Cancer", PLOS ONE, vol. 8, no. 4, 8 April 2013 (2013-04-08), page e60359, XP055693556, DOI: 10.1371/journal.pone.0060359

## Description

### FIELD OF THE INVENTION

The invention relates to methods allowing the delivery of human HTRA1 protein and its variants into mammalian cells, tissues or organisms without using transfection reagents. HTRA1 variants include but are not restricted to coupled cargo proteins for complementation, activation or inhibition of cellular pathways for basic and translational research as well as for therapy.

### BACKGROUND OF THE INVENTION

### Protein delivery into mammalian cells

Precise and graded manipulation of protein levels in mammalian cells is an unsolved problem in basic biological and clinical research as well as in clinical application. The current state of the art includes genetic manipulation, the use of cell penetrating peptides, nanoparticles or by lipophilic transfection reagents. Genetic manipulation uses e.g. transfection of expression plasmids or gene editing. The drawbacks of both are the limited tuneability of expression levels and side effects resulting from the genetic manipulation. These side effects include integration of the plasmids into the chromosome leading to gene disruption or activation as well as the manifestation of additional (i.e. suppressor) mutations. On the other hand, cell penetrating peptides (CPP) are able to deliver the cargo without genetic manipulation (Bechara and Sagan, 2013). The same is true for nanoparticles where proteins are either encapsulated or surface-attached as well as for lipophilic transduction reagents (Hamidi et al., 2007). However, these techniques have significant drawbacks in their efficiency, cytotoxicity and convenience. Other limitations include the risk of altering the biological activity of the cargo when covalently attached to cell penetrating peptides. Moreover, endosomal escape remains a major limitation and the rate-limiting step of CPP-mediated drug delivery (Shete et al., 2014).

### HtrA family of serine proteases

The HtrA family represents a unique class of oligomeric serine proteases (Clausen et al., 2011). Their defining feature is the combination of a catalytic domain with one or more C-terminal PDZ domains. PDZ domains are protein modules that mediate specific protein-protein interactions and bind preferentially to the C-terminal 3-4 residues of the target protein. The PDZ domains of HtrA proteases are involved in several key structural and functional aspects. They sense protein folding stress by binding the C-termini of misfolded and mislocalised proteins. Peptide binding leads to activation of the proteolytic activity by an allosteric mechanism the mechanism of which is known at atomic resolution.

### Structural features of HtrA proteases

The protease domain of all HtrAs adopts the chymotrypsin fold that consists of two six-stranded β-barrel sub-domains. The active site cleft is located at the interface of two perpendicularly arranged barrel domains and is constructed by several loops of both. The C-terminal PDZ domain(s) of HtrA proteases share similarities with many other protein-protein interaction modules in that they are small, compact, and globular domains and their N- and C-termini are in close proximity to one another. The PDZ fold consists of six β-strands forming two β-sheets, and two α-helices that cap their edges. Compared with the fold of PDZ domains present in eukaryotic scaffolding proteins, the PDZ domains of HtrA proteases have a circular permutation, in which the N- and C-terminal strands are exchanged. Consequently, the carboxylate-binding loop, a conserved PDZ sequence often referred to as GLGF loop, is the immediate N-terminus of the domain that is connected by a short linker to the protease domain in HtrA proteins. The carboxylate-binding loop is followed by β-strand A which forms, together with helix αB, the canonical-peptide binding site of the PDZ domain. βA is instrumental to anchor the peptide in a β-augmentation process, whereas the binding pocket for the C-terminal residue determines substrate specificity, selecting for peptides with a hydrophobic C-terminal residue. Compared with classic PDZ domains, the PDZ domain of HtrA proteases harbours several additional structural features that are important for oligomer assembly and cellular localization.

### Controlled activation of HtrA proteases

HtrA proteases exist as oligomers, thus allowing communication between adjacent subunits to regulate protease function in a reversible and tightly controlled manner. The activation cascade is initiated by the sensor loop L3, which specifically rearranges upon perceiving a distinct biological signal. The repositioned loop L3 can now interact with the activation loop LD of a neighbouring protomer in the HtrA trimer, thereby inducing the proper adjustment of the activation domain. The construction of this domain, which is composed of the active site loops L1, L2 and LD, as well as its disorder-order transition upon activation, is conserved between trypsin-like and HtrA proteases, and highlights a common catalytic mechanism. However, in HtrA proteases, the switch in activity is directly mediated by the ligand-dependent interaction of loops L3 and LD rather than by propeptide cleavage during zymogen activation and is thus reversible. Moreover, activation of the oligomeric HtrA proteases is often a highly cooperative process, as shown for DegS and DegP (Clausen et al., 2011).

### Human HTRA1

The ubiquitously expressed human HTRA1 consists of a signal sequence for secretion, a partial insulin like growth factor binding protein-7 (IGFBP)-7 domain, a serine protease domain and one C-terminal PDZ domain. Like all other HtrAs, HTRA1 switches reversibly between active and inactive conformations (Truebestein et al., 2011). The PDZ domain of HTRA1 is thought to mediate e.g. cellular localization by binding to specific interaction partners (Chien et al., 2009).

HTRA1 has at least three cellular localizations. Extracytoplasmic HTRA1 is involved in the homeostasis of the extracellular matrix (Clausen et al., 2011) and intracellular HTRA1 was localized to the cytoplasm (Campioni et al., 2010) to microtubules and to the nucleus (Chien et al., 2009; Clawson et al., 2008). Microtubule-associated HTRA1 degrades tubulins thereby inhibiting cell migration (Clausen et al., 2011). Moreover, HTRA1 has been implicated in several severe pathologies including cancer, agerelated macular degeneration, Alzheimer's disease (AD), arthritis and familial ischemic cerebral small-vessel disease (Clausen et al., 2011). In these diseases, protein fragments or aggregates are either causative for disease or are disease modifying factors that are produced or degraded by HTRA1.

### The tau protein

The microtubule-associated tau protein aggregates into intracellular neurofibrillary tangles representing one hallmark of AD and other tauopathies. Normal tau is thought to regulate microtubule dynamics. Interaction of tau with microtubules is mediated by its microtubule binding domain (MTBD) composed of several repeats sharing the consensus sequence VxSKxGSxxN(L/I)xHxPGGG. Free tau protein, typically resulting from hyperphosphorylation, polymerises into straight or paired helical filaments (PHF), ribbons and other conformations. The core domain of PHFs consists of three or four repeats of MTBD mediating tau-tau interactions. In addition, proteolytic processing of tau stimulates the assembly of tau into fibrils. Such assembly can capture further full-length tau proteins causing progressive growth of aggregates ultimately causing cell death and therefore pathological features (for review see (Spillantini and Goedert, 2013)).

In view of the above, there was a need in the art to control the activity of HTRA1 in cells, in particular for research purposes to further elucidate the role of HTRA1 and as therapeutic means in the treatment of diseases involving aberrant HTRA1 activity.

### SUMMARY OF THE INVENTION

The present inventors have found that cells spontaneously take up HTRA1 without the need of transfection reagents. When cells are incubated in the presence of HTRA1, in particular the HTRA1 protease domain, it is rapidly transferred into the cells. Hence, the amount of HTRA1 in the cells can be increased by adding HTRA1 to the surrounding medium. The HTRA1 activity in the cells can be further controlled by using HTRA1 variants having a modulated activity such as constitutively active or dominant negative variants. It is even possible to use HTRA1 for transferring cargo such as other proteins across the cell membrane into the cytosol of cells. Therefore, HTRA1 can be employed as carrier protein for other molecules. Due to the high similarity between HTRA1 and the other HTRA family members, in particular HTRA3 and HTRA4, these HTRA proteins can also be used for respective applications.

The present invention provides in a first aspect a method for modulating the HTRA1 activity inside a cell, comprising the steps of
(a) contacting the cell with a HTRA1 polypeptide in the absence of any other means suitable for inducing uptake of polypeptides into a cell;
(b) incubating the cell in the presence of the HTRA1 polypeptide for a period of time sufficient for uptake of the HTRA1 polypeptide into the cell;
wherein the cell is a mammalian cell; and wherein the HTRA1 polypeptide comprises the HTRA1 protease domain or an amino acid sequence derived therefrom which shares an identity with the amino acid sequence of the HTRA1 protease domain over its entire length of at least 80%.

In a second aspect, the present invention provides a HTRA1 polypeptide for use in the treatment of a disease in a human being which benefits from increasing the HTRA1 activity, wherein a pharmaceutical preparation containing the HTRA1 polypeptide is locally applied to the site of the disease in order to modulate the HTRA1 activity inside the affected cells, wherein the pharmaceutical preparation does not contain any other means suitable for inducing uptake of polypeptides into a cell, wherein the HTRA1 polypeptide has protease activity and comprises the HTRA1 protease domain or an amino acid sequence derived therefrom which shares an identity with the amino acid sequence of the HTRA1 protease domain over its entire length of at least 80%; and wherein
(i) the disease is a tauopathy such as Alzheimer's disease and the HTRA1 polypeptide is locally applied to the brain; or
(ii) the disease is cancer comprising cancer cells having a decreased HTRA1 activity and the HTRA1 polypeptide is locally applied to the tumor site.

In a third aspect of the present invention an in vitro method for transport of a molecule of interest into a cell is provided, comprising the steps of
(a) providing a conjugate of the molecule of interest and a HTRA1 polypeptide;
(b) contacting the cell with the conjugate;
(c) incubating the cell in the presence of the conjugate for a period of time sufficient for uptake of the conjugate into the cell;
wherein the HTRA1 polypeptide comprises the HTRA1 protease domain or an amino acid sequence derived therefrom which shares an identity with the amino acid sequence of the HTRA1 protease domain over its entire length of at least 80%.

In a fourth aspect, the present invention pertains to a conjugate comprising a therapeutic agent and a HTRA1 polypeptide, wherein the HTRA1 polypeptide comprises the HTRA1 protease domain or an amino acid sequence derived therefrom which shares an identity with the amino acid sequence of the HTRA1 protease domain over its entire length of at least 80%.

In a fifth aspect the present invention is directed to a conjugate comprising a therapeutic agent and the HTRA1 polypeptide according to the fourth aspect for use in the treatment of a disease, wherein a pharmaceutical preparation containing the conjugate is locally applied to the site of the disease.

Other objects, features, advantages and aspects of the present invention will become apparent to those skilled in the art from the following description and appended claims.

### DEFINITIONS

As used herein, the following expressions are generally intended to preferably have the meanings as set forth below, except to the extent that the context in which they are used indicates otherwise.

The expression "comprise", as used herein, besides its literal meaning also includes and specifically refers to the expressions "consist essentially of" and "consist of". Thus, the expression "comprise" refers to embodiments wherein the subject-matter which "comprises" specifically listed elements does not comprise further elements as well as embodiments wherein the subject-matter which "comprises" specifically listed elements may and/or indeed does encompass further elements. Likewise, the expression "have" is to be understood as the expression "comprise", also including and specifically referring to the expressions "consist essentially of" and "consist of".

A "member of the HTRA family" as used herein refers to human HTRA1, human HTRA3 and human HTRA4 and any isoforms or variants thereof as well as any homologues thereof in other species. In certain embodiments, the member of the HTRA family specifically refers to HTRA1. The term "HTRA polypeptide" refers to a HTRA1 polypeptide, a HTRA3 polypeptide or a HTRA4 polypeptide or to a mixture of one or more of these polypeptides. In particular, a HTRA polypeptide is a HTRA1 polypeptide.

The term "HTRA1" as used herein in particular refers to the serine protease HTRA1, including the precursor protein containing the N terminal signal peptide and the processed mature protein without the signal peptide. The HTRA1 may be from any species, but in particular is human HTRA1. In specific embodiments, HTRA1 has the amino acid sequence according to any one of SEQ ID NOs: 1 to 3, in particular the amino acid sequence of SEQ ID NO: 1, or an amino acid sequence derived therefrom. In further embodiments, HTRA1 has the amino acid sequence according to amino acid positions 23 to 480 of any one of SEQ ID NOs: 1 to 3, in particular the amino acid sequence according to amino acid positions 23 to 480 of SEQ ID NO: 1, or an amino acid sequence derived therefrom. A "HTRA1 polypeptide" is a polypeptide derived from HTRA1 or a part thereof. In certain embodiments, the HTRA1 polypeptide comprises the protease domain of HTRA1, in particular an amino acid sequence according to amino acid positions 158 to 373 of any one of SEQ ID NOs: 1 to 3, especially SEQ ID NO: 1, or an amino acid sequence derived therefrom. HTRA1 and a HTRA1 polypeptide may have a protease activity, but do not need to have a protease activity. The term "HTRA1 activity" as used herein in particular refers to a protease activity, especially to the activity to proteolytically cleave tau proteins, tubulins or extracellular matrix proteins such as fibronectin.

The term "HTRA3" as used herein in particular refers to the serine protease HTRA3, including the precursor protein containing the N terminal signal peptide and the processed mature protein without the signal peptide. The HTRA3 may be from any species, but in particular is human HTRA3. In specific embodiments, HTRA3 has the amino acid sequence according to SEQ ID NO: 4, or an amino acid sequence derived therefrom. In further embodiments, HTRA3 has the amino acid sequence according to amino acid positions 18 to 453 of SEQ ID NO: 4, or an amino acid sequence derived therefrom. A "HTRA3 polypeptide" is a polypeptide derived from HTRA3 or a part thereof. In certain embodiments, the HTRA3 polypeptide comprises the protease domain of HTRA3, in particular an amino acid sequence according to amino acid positions 132 to 350 of SEQ ID NO: 4, or an amino acid sequence derived therefrom. HTRA3 and a HTRA3 polypeptide may have a protease activity, but do not need to have a protease activity. The term "HTRA3 activity" as used herein in particular refers to a protease activity, especially to the activity to proteolytically cleave beta-casein or extracellular matrix proteins such as fibronectin.

The term "HTRA4" as used herein in particular refers to the serine protease HTRA4, including the precursor protein containing the N terminal signal peptide and the processed mature protein without the signal peptide. The HTRA4 may be from any species, but in particular is human HTRA4. In specific embodiments, HTRA4 has the amino acid sequence according to SEQ ID NO: 5, or an amino acid sequence derived therefrom. In further embodiments, HTRA4 has the amino acid sequence according to amino acid positions 32 to 476 of SEQ ID NO: 5, or an amino acid sequence derived therefrom. A "HTRA4 polypeptide" is a polypeptide derived from HTRA4 or a part thereof. In certain embodiments, the HTRA4 polypeptide comprises the protease domain of HTRA4, in particular an amino acid sequence according to amino acid positions 156 to 371 of SEQ ID NO: 5, or an amino acid sequence derived therefrom. HTRA4 and a HTRA4 polypeptide may have a protease activity, but do not need to have a protease activity. The term "HTRA4 activity" as used herein in particular refers to a protease activity.

A target amino acid sequence is "derived" from a reference amino acid sequence, for example, if the target amino acid sequence shares a homology or identity with the reference amino acid sequence over its entire length or with a corresponding part of the reference amino acid sequence over its entire length of at least 50%, preferably at least 60%, at least 70%, at least 75%, more preferably at least 80%, at least 85%, at least 90%, at least 93%, at least 95% or at least 97%. In particular embodiments, a target amino acid sequence which is "derived" from a reference amino acid sequence is 100% homologous, or in particular 100% identical, to the reference amino acid sequence over its entire length or to a corresponding part of the reference amino acid sequence over its entire length.

"Specific binding" preferably means that an agent such as a PDZ binding peptide binds stronger to a target such as a PDZ domain for which it is specific compared to the binding to another target. An agent binds stronger to a first target compared to a second target if it binds to the first target with a dissociation constant (Kd) which is lower than the dissociation constant for the second target. Preferably the dissociation constant for the target to which the agent binds specifically is more than 2-fold, preferably more than 5-fold, more preferably more than 10-fold, even more preferably more than 20-fold, 50-fold, 100-fold, 200-fold, 500-fold or 1000-fold lower than the dissociation constant for the target to which the agent does not bind specifically.

As used herein, the term "polypeptide" refers to a molecular chain of amino acids attached to each other via peptide bonds. A polypeptide can contain any of the naturally occurring amino acids as well as artificial amino acids and can be of biologic or synthetic origin. A polypeptide may be modified, naturally (post-translational modifications) or synthetically, by e.g. glycosylation, amidation, carboxylation and/or phosphorylation. A polypeptide comprises at least two amino acids, but does not have to be of any specific length; this term does not include any size restrictions. Preferably, a polypeptide comprises at least 10 amino acids, preferably at least 50 amino acids, more preferred at least 80 amino acids and most preferred at least 100 amino acids.

The term "nucleic acid" includes single-stranded and double-stranded nucleic acids and ribonucleic acids as well as deoxyribonucleic acids. It may comprise naturally occurring as well as synthetic nucleotides and can be naturally or synthetically modified, for example by methylation, 5'- and/or 3'-capping.

The term "conjugate" particularly means two or more compounds which are linked together so that at least some of the properties from each compound are retained in the conjugate. Linking may be achieved by a covalent or non-covalent bond. Preferably, the compounds of the conjugate are linked via a covalent bond. The different compounds of a conjugate may be directly bound to each other via one or more covalent bonds between atoms of the compounds. Alternatively, the compounds may be bound to each other via a linker molecule wherein the linker is covalently attached to atoms of the compounds. If the conjugate is composed of more than two compounds, then these compounds may, for example, be linked in a chain conformation, one compound attached to the next compound, or several compounds each may be attached to one central compound.

The term "patient" means according to the invention a human being, a nonhuman primate or another animal, in particular a mammal such as a cow, horse, pig, sheep, goat, dog, cat or a rodent such as a mouse and rat. In a particularly preferred embodiment, the patient is a human being.

The term "cancer" according to the invention in particular comprises leukemias, seminomas, melanomas, teratomas, lymphomas, neuroblastomas, gliomas, rectal cancer, endometrial cancer, kidney cancer, adrenal cancer, thyroid cancer, blood cancer, skin cancer, cancer of the brain, cervical cancer, intestinal cancer, liver cancer, colon cancer, stomach cancer, intestine cancer, head and neck cancer, gastrointestinal cancer, lymph node cancer, esophagus cancer, colorectal cancer, pancreas cancer, ear, nose and throat (ENT) cancer, breast cancer, prostate cancer, cancer of the uterus, ovarian cancer and lung cancer and the metastases thereof. Examples thereof are lung carcinomas, mamma carcinomas, prostate carcinomas, colon carcinomas, renal cell carcinomas, cervical carcinomas, or metastases of the cancer types or tumors described above. The term cancer according to the invention also comprises cancer metastases. By "tumor" is meant a group of cells or tissue that is formed by misregulated cellular proliferation. Tumors may show partial or complete lack of structural organization and functional coordination with the normal tissue, and usually form a distinct mass of tissue, which may be either benign or malignant.

By "metastasis" is meant the spread of cancer cells from its original site to another part of the body. The formation of metastasis is a very complex process and normally involves detachment of cancer cells from a primary tumor, entering the body circulation and settling down to grow within normal tissues elsewhere in the body. When tumor cells metastasize, the new tumor is called a secondary or metastatic tumor, and its cells normally resemble those in the original tumor. This means, for example, that, if breast cancer metastasizes to the lungs, the secondary tumor is made up of abnormal breast cells, not of abnormal lung cells. The tumor in the lung is then called metastatic breast cancer, not lung cancer.

The term "pharmaceutical composition" particularly refers to a composition suitable for administering to a human or animal, i.e., a composition containing components which are pharmaceutically acceptable. Preferably, a pharmaceutical composition comprises an active compound or a salt or prodrug thereof together with a carrier, diluent or pharmaceutical excipient such as buffer, preservative and tonicity modifier.

### DETAILED DESCRIPTION OF THE INVENTION

The introduction of human HTRA1 into mammalian cells is generally useful for cell biological experiments. The benefits of this technology are that the levels of HTRA1 introduced into cells are tuneable and homogenous, i.e. the level of HTRA1 concentrations is the same in all cells of a population. Furthermore, delivery of HTRA1 with altered or different PDZ domains into mammalian cells will cause association of this protease or its derivatives with specific cellular compartments or proteins or protein complexes causing a change in substrates that are targeted by HTRA1. This technology allows the use of HTRA1 as a tool to manipulate specific targets. HTRA1 mutants delivered into mammalian cells or tissues or bodies without using transfection reagents are able promote the formation of heterooligomers of HTRA1 with altered function and activity.

Heterooligomer formation between HTRA1 variants allows tuning of the activity and function of HTRA1. This is useful for manipulation of experimental systems including e.g. patient samples addressing the biological function of HTRA1. Moreover, modified HTRA1 might be delivered to patients to treat diseases that are caused or modified by HTRA1. Explicitly, diseases caused or modified by elevated HTRA1 levels could be treated by exogenous application of HTRA1 variants causing a reduction of HTRA1's activity e.g. by applying proteolytically inactive HTRA1 variants or HTRA1 variants that are dominant negative by e.g. interfering with folding or assembly of HTRA1 oligomers and or the stability of the heterooligomers. Diseases caused or modified by low levels of HTRA1 could be treated by exogenous application of either wildtype HTRA1 or HTRA1 variants with elevated activity or by exogenous application of constitutively active HTRA1 variants or HTRA1 variants causing increased stability of heterooligomeric HTRA1.

Moreover, HTRA1 can be used as transport agent ("carrier") to deliver cargo into mammalian cells. The cargo can be fused to the primary amino acid sequence of HTRA1. In this case, the hybrid proteins can stay intact or can be proteolysed upon entry into cells by cellular proteases cleaving the linker that connects HTRA1 and its cargo. This proteolytic processing allows the cargo to detach from HTRA1 to move to a cellular localisation of choice e.g. by exposing a specific cellular localisation signal. Alternatively, the cargo can be non-covalently coupled to HTRA1 by binding to the PDZ domain of HTRA1. This non-covalent interaction allows dissociation of cargo from HTRA1 without proteolytic processing.

Delivery of cargos to mammalian cells, tissues or bodies is useful to study diseases caused or modified by the cargo proteins. Also, delivery of cargo such as derivatives or inhibitors of specific target proteins to patients can be used to treat diseases that are caused or modified by said target proteins.

In view of the close resemblance between HTRA1 and HTRA3 and HTRA4, these other members of the HTRA family can likewise be used to modulate the respective HTRA activity in cells or to transport cargo into cells. In particular, HTRA3 and HTRA4 share a sequence identity of more than 50% to HTRA1 on the amino acid level, which is a very close relatedness in naturally occurring proteins.

### The method for modulating the HTRA activity in a cell

The present invention provides in a first aspect a method for modulating the HTRA1 activity inside a cell, comprising the steps of
(a) contacting the cell with a HTRA1 polypeptide in the absence of any other means suitable for inducing uptake of polypeptides into a cell;
(b) incubating the cell in the presence of the HTRA1 polypeptide for a period of time sufficient for uptake of the HTRA1 polypeptide into the cell;
wherein the cell is a mammalian cell; and wherein the HTRA1 polypeptide comprises the HTRA1 protease domain or an amino acid sequence derived therefrom which shares an identity with the amino acid sequence of the HTRA1 protease domain over its entire length of at least 80%.

The HTRA1 polypeptide used in the method for modulating the HTRA1 activity in a cell in particular has the ability to facilitate uptake into a cell. Especially, the HTRA1 polypeptide comprises the protease domain of HTRA1 or an amino acid sequence derived therefrom.

In certain embodiments, modulating the HTRA1 activity in a cell means increasing the HTRA1 activity in the cell. In these embodiments, the HTRA1 polypeptide preferably has protease activity. The protease activity of the HTRA1 polypeptide may be lower than the protease activity of wildtype HTRA1, similar thereto or even higher. In particular, the protease activity of the HTRA1 polypeptide is higher than that of wildtype HTRA1. In a specific embodiment, the HTRA1 polypeptide is a constitutively active HTRA1 variant.

In particular, the HTRA1 polypeptide may comprise the HTRA1 protease domain or an amino acid sequence derived therefrom, such as the amino acid sequence according to amino acid positions 158 to 373 of SEQ ID NO: 1, 2 or 3, in particular of SEQ ID NO: 1, or an amino acid sequence derived therefrom. In certain embodiments, the HTRA1 polypeptide may comprises the protease domain and the PDZ domain of HTRA1 or an amino acid sequence derived therefrom, in particular the amino acid sequence according to amino acid positions 158 to 480 of SEQ ID NO: 1, 2 or 3, in particular of SEQ ID NO: 1, or an amino acid sequence derived therefrom. In further embodiments, the HTRA1 polypeptide may comprises the entire HTRA1, in particular full length mature HTRA1, or an amino acid sequence derived therefrom, especially the amino acid sequence according to amino acid positions 23 to 480 of SEQ ID NO: 1, 2 or 3, in particular of SEQ ID NO: 1, or an amino acid sequence derived therefrom.

In certain embodiments, modulating the HTRA1 activity in a cell means decreasing the HTRA1 activity in the cell. In these embodiments, the HTRA1 polypeptide preferably does not have any protease activity or has a reduced protease activity in comparison to wildtype HTRA1. In particular, in the HTRA1 polypeptide the amino acid residue corresponding to serine at position 328 of SEQ ID NO: 1 is any amino acid residue except serine, such as for example alanine. In specific embodiments, the HTRA1 polypeptide is a dominant negative HTRA1 variant. For example, a dominant negative HTRA1 variant may have a reduced ability to form trimers.

In specific embodiments, the HTRA1 polypeptide comprises a cellular localization signal. Suitable examples of such cellular localization signals are nuclear, peroxisomal or mitochondrial localization sequences. Exemplary cellular localization signals are described herein below.

In a first step, the HTRA1 polypeptide is contacted with the cell or cells in which the HTRA1 activity is to be modulated. Uptake of the HTRA1 polypeptide into the cells is facilitated by the HTRA1 polypeptide itself. In particular, no other means are necessary for uptake of the HTRA1 polypeptide into the cells. Therefore, the first step is performed in the absence of any other means suitable for inducing uptake of polypeptides into a cell. Such other means in particular include cell penetrating peptides, nanoparticles and lipophilic transduction reagents. Contacting the cells with the HTRA1 polypeptide is achieved, for example, by adding the HTRA1 polypeptide to the cell environment, for example into the cell medium.

In certain embodiments, the cell is contacted with the HTRA1 polypeptide in a concentration of at least 1 µg/ml. Specifically, the HTRA1 polypeptide is used in a concentration of at least 5 µg/ml, at least 10 µg/ml, at least 20 µg/ml, at least 50 µg/ml, or at least 100 µg/ml. The HTRA1 polypeptide may in particular be used in a concentration in the range of from 1 µg/ml to 1000 µg/ml, especially from 10 µg/ml to 150 µg/ml. In further embodiments, the cell is contacted with the HTRA1 polypeptide in a concentration of at least 0.05 µM. Specifically, the HTRA1 polypeptide is used in a concentration of at least 0.2 µM, at least 0.5 µM, at least 1 µM, at least 2 µM, or at least 5 µM. The HTRA1 polypeptide may in particular be used in a concentration in the range of from 0.05 µM to 50 µM, especially from 0.5 µM to 5 µM.

In the second step, the cell is incubated in the presence of the HTRA1 polypeptide. The time of incubation is chosen so as to be sufficient for uptake into the cells of at least a part of the HTRA1 polypeptides contacted with the cells. In particular, the amount of HTRA1 polypeptides taken up by the cells after the incubation step should be sufficient to achieve the desired modulation of HTRA1 activity in the cells. In certain embodiments, the cell is incubated in the presence of the HTRA1 polypeptide for at least 1 min, in particular at least 5 min, at least 10 min, at least 30 min or at least 60 min. The cell may in particular be incubated in the presence of the HTRA1 polypeptide for a period of time in the range of from 1 min to 120 min, especially 10 min to 60 min.

In certain embodiments, the method for modulating the HTRA1 activity in a cell is not performed in the human body and in particular not performed in the human or animal body. Specifically, the method for modulating the HTRA1 activity in a cell is an in vitro method.

Furthermore, a method for modulating the HTRA3 activity in a cell is described, comprising the steps of
(a) contacting the cell with a HTRA3 polypeptide in the absence of any other means suitable for inducing uptake of polypeptides into a cell;
(b) incubating the cell in the presence of the HTRA3 polypeptide for a period of time sufficient for uptake of the HTRA3 polypeptide into the cell.

In addition, a method for modulating the HTRA4 activity in a cell is described, comprising the steps of
(a) contacting the cell with a HTRA4 polypeptide in the absence of any other means suitable for inducing uptake of polypeptides into a cell;
(b) incubating the cell in the presence of the HTRA4 polypeptide for a period of time sufficient for uptake of the HTRA4 polypeptide into the cell.

The features and embodiments described above with respect to HTRA1 and the HTRA1 polypeptide for the method for modulating the HTRA1 activity in a cell likewise apply to HTRA3 and the HTRA3 polypeptide and HTRA4 and the HTRA4 polypeptide.

Furthermore, in embodiments where the HTRA3 polypeptide comprises the HTRA3 protease domain or an amino acid sequence derived therefrom, the HTRA3 polypeptide in particular comprises the amino acid sequence according to amino acid positions 132 to 350 of SEQ ID NO: 4, or an amino acid sequence derived therefrom. In certain embodiments, the HTRA3 polypeptide may comprises the protease domain and the PDZ domain of HTRA3 or an amino acid sequence derived therefrom, in particular the amino acid sequence according to amino acid positions 132 to 453 of SEQ ID NO: 4 or an amino acid sequence derived therefrom. In further embodiments, the HTRA3 polypeptide may comprises the entire HTRA3, in particular full length mature HTRA3, or an amino acid sequence derived therefrom, especially the amino acid sequence according to amino acid positions 18 to 453 of SEQ ID NO: 4, or an amino acid sequence derived therefrom.

In embodiments where the HTRA4 polypeptide comprises the HTRA4 protease domain or an amino acid sequence derived therefrom, the HTRA4 polypeptide in particular comprises the amino acid sequence according to amino acid positions 156 to 371 of SEQ ID NO: 5, or an amino acid sequence derived therefrom. In certain embodiments, the HTRA4 polypeptide may comprises the protease domain and the PDZ domain of HTRA4 or an amino acid sequence derived therefrom, in particular the amino acid sequence according to amino acid positions 156 to 476 of SEQ ID NO: 5 or an amino acid sequence derived therefrom. In further embodiments, the HTRA4 polypeptide may comprises the entire HTRA4, in particular full length mature HTRA4, or an amino acid sequence derived therefrom, especially the amino acid sequence according to amino acid positions 32 to 476 of SEQ ID NO: 5, or an amino acid sequence derived therefrom.

In embodiments where the HTRA3 polypeptide does not have any protease activity or has a reduced protease activity in comparison to wildtype HTRA3, the amino acid residue corresponding to serine at position 305 of SEQ ID NO: 4 in particular is any amino acid residue except serine, such as for example alanine, in the HTRA3 polypeptide.

In embodiments where the HTRA4 polypeptide does not have any protease activity or has a reduced protease activity in comparison to wildtype HTRA4, the amino acid residue corresponding to serine at position 326 of SEQ ID NO: 5 in particular is any amino acid residue except serine, such as for example alanine, in the HTRA4 polypeptide.

### The therapeutic use of HTRA polypeptides

The automatic uptake of HTRA1, HTRA3 and HTRA4 into cells without the need of other means for inducing said uptake can also be used for therapeutic applications. In particular, several diseases are known wherein HTRA1, HTRA3 or HTRA4 plays a crucial role. Patients having such a disease would benefit from modulation of the activity of the respective member of the HTRA family in affected cells, e.g. an increase of HTRA1 activity in diseases involving a reduced HTRA1 activity and a decrease in HTRA1 activity in diseases involving a HTRA1 activity which is too high.

Hence, a HTRA1 polypeptide may be used in the treatment of a disease which benefits from modulating the HTRA1 activity. This treatment includes locally applying a pharmaceutical preparation containing the HTRA1 polypeptide to the site of the disease. In particular, the pharmaceutical preparation does not contain any other means suitable for inducing uptake of polypeptides into a cell.

In certain embodiments, the patient having the disease benefits from reducing the HTRA1 activity. In these embodiments, the HTRA1 polypeptide preferably is a dominant negative HTRA1 variant, especially a dominant negative HTRA1 variant which has a reduced ability to form trimers. In specific embodiments, the HTRA1 polypeptide does not have a protease activity. Exemplary diseases wherein a reduced HTRA1 activity is beneficial are selected from the group consisting of macular degeneration, osteoarthritis, reumathoid arthritis, osteoporosis, intervertebral disc degeneration and cancer. The cancer is in particular a cancer comprising cancer cells having an increased HTRA1 activity compared to normal cells of the same tissue, such as an increase HTRA1 expression level, for example cancer cells with HTRA1 overexpression. Examples of such cancers include fast migrating and invading gliomas and papillary thyroid carcinomas.

In other embodiments, the patient having the disease benefits from increasing the HTRA1 activity. In these embodiments, the HTRA1 polypeptide preferably has protease activity, and especially is a constitutively active HTRA1 variant. Exemplary diseases wherein an increased HTRA1 activity is beneficial are selected from the group consisting of tauopathies such as Alzheimer's disease, CARASIL (cerebral autosomal recessive arteriopathy with subcortical infarcts and leukoencephalopathy), ischemic cerebral small-vessel disease and cancer. The cancer is in particular a cancer comprising cancer cells having a decreased HTRA1 activity compared to normal cells of the same tissue, such as cancer cells expressing mutated HTRA1 with reduced or lacking protease activity or cancer cells having a decrease HTRA1 expression level, for example cancer cells which do not express HTRA1. Examples of such cancers include gastric carcinomas, breast cancer, metastatic esophageal carcinomas, urothelial bladder cancer, endometrial cancer, hepatocellular carcinomas, and ovarian cancer.

The HTRA1 polypeptide in particular is a HTRA1 polypeptide as described herein.

The HTRA1 polypeptide is locally applied, especially to the site of the disease, in order to modulate the HTRA1 activity in the affected cells. The HTRA1 polypeptide may be applied topically, by injection or by eye drops, depending on the site of the disease. For example,
(i) the disease is a tauopathy such as Alzheimer's disease and the HTRA1 polypeptide is locally applied to the brain;
(ii) the disease is cancer and the HTRA1 polypeptide is locally applied to the tumor site;
(iii) the disease is macular degeneration and the HTRA1 polypeptide is locally applied to the eye;
(iv) the disease is arthritis and the HTRA1 polypeptide is locally applied to the affected joints;
(v) the disease is osteoporosis and the HTRA1 polypeptide is locally applied to the affected bones; or
(vi) the disease is CARASIL or ischemic cerebral small-vessel disease and the HTRA1 polypeptide is locally applied to the brain.

The present application further describes a method for treatment of a disease, comprising administering a HTRA1 polypeptide to a patient in need thereof. This treatment includes locally applying a pharmaceutical preparation containing the HTRA1 polypeptide to the site of the disease. The patient benefits from modulating the HTRA1 activity at the site of the disease, either by increasing or by decreasing the HTRA1 activity. In particular, the pharmaceutical preparation does not contain any other means suitable for inducing uptake of polypeptides into a cell. The features and embodiments described above with respect to the HTRA1 polypeptide for use in the treatment of a disease also apply likewise to the method for treatment of a disease.

In further embodiments, the present application describes a HTRA3 polypeptide for use in the treatment of a disease which benefits from modulating the HTRA3 activity. This treatment includes locally applying a pharmaceutical preparation containing the HTRA3 polypeptide to the site of the disease. In particular, the pharmaceutical preparation does not contain any other means suitable for inducing uptake of polypeptides into a cell.

In even further embodiments, the present application describes a HTRA4 polypeptide for use in the treatment of a disease which benefits from modulating the HTRA4 activity. This treatment includes locally applying a pharmaceutical preparation containing the HTRA4 polypeptide to the site of the disease. In particular, the pharmaceutical preparation does not contain any other means suitable for inducing uptake of polypeptides into a cell.

The features and embodiments described above with respect to HTRA1 and the HTRA1 polypeptide for its use in the treatment of a disease likewise apply to HTRA3 and the HTRA3 polypeptide and HTRA4 and the HTRA4 polypeptide.

Diseases wherein the activity of HTRA3 and/or HTRA4 is too high or too low in the affected cells include, for example, cancer, infertility or pregnancy disorders such as pre-eclampsia and intra uterine growth.

### The method for transport of a molecule of interest into a cell

In a third aspect, the present invention provides an in vitro method for transport of a molecule of interest into a cell, comprising the steps of
(a) providing a conjugate of the molecule of interest and a HTRA1 polypeptide;
(b) contacting the cell with the conjugate;
(c) incubating the cell in the presence of the conjugate for a period of time sufficient for uptake of the conjugate into the cell.
wherein the HTRA1 polypeptide comprises the HTRA1 protease domain or an amino acid sequence derived therefrom which shares an identity with the amino acid sequence of the HTRA1 protease domain over its entire length of at least 80%.

With the present invention, the inventors established a novel uptake system for proteins and other molecules ("cargo") into mammalian cells, namely the method for transport of a molecule of interest into a cell as described herein. The "cargo" which is to be transported into the cells can be any molecule of interest, including in particular proteins, polypeptides, nucleic acids, and therapeutically active substances. Complexes of two or more separate molecules can be transported, too. The uptake system in particular may have the following properties:
a) The cells are homogenously targeted. HTRA1 and its cargo are taken up homogenously within a population of cells.
b) The concentration of internalised protein is tuneable. Titration experiments show that the levels of internalized HTRA1 and its cargo correlates with the amounts of protein added to cells. Therefore, the levels of HTRA1 and its cargo are tuneable via titration of exogenously applied HTRA1 or HTRA1-cargo.
c) The internalised protein is stable. Data indicate stability of the recombinant proteins correlate with the stability of the native protein.
d) The internalised protein has biological activity. Internalised HTRA1 is biologically active as tau fibrils are degraded. Also, the activity of cargos such as GFP can be detected within cells.
e) The internalised protein is targeted to a specific cellular compartment. The HTRA1 delivery vehicle can be modified in various ways to reach these aims. For example, HTRA1 lacking its PDZ domain is homogenously distributed in the cytosol; HTRA1 containing its PDZ domain is located to the same subcompartments as native HTRA1; and the presence of compartment specific targeting signals in the cargo proteins lead to specific cellular localisation of cargo after release from HTRA1.
f) Internalisation of mutant HTRA1 leads to heterooligomer formation with native HTRA1 causing modulated HTRA1 activity. Here, mutant forms of HTRA1 including mutants that have reduced, increased or dominant negative or dominant positive activity could be internalised. Following internalisation, these HTRA1 mutants would interact with native wt HTRA1 in native samples or mutant HTRA1 in patient samples or patient tissue, leading to heterooligomer fomation. These heterooligomers would have altered activities and/or functions compared to the HTRA1 proteins originally present in the samples.
g) Delivery of polypeptide cargos can be achieved by three main mechanisms into cells.

First, the cargo can be genetically fused to HTRA1. Here, a gene fusion is constructed between e.g. the gene fragment encoding the protease domain of HTRA1 and the gene fragment encoding the cargo. The gene fusion is constructed such that the two gene fragments are present in the same reading frame, encoding a hybrid protein composed of HTRA1 and the cargo.

Second, carrier (i.e. HTRA1) and cargo are coupled via a disulfide bond. Upon internalisation into the cytosol that is a reducing environment the disulfide bond is reduced leading to dissociation of carrier and cargo. Dissociation would allow the cargo to move to a cellular localisation that is different from the localisation of the carrier. It is an advantage that HTRA1 lacking its N-terminal domain does not contain Cys residues. Should the cargo contain Cys residues, these could be exchanged e.g. by Ser or other suitable residues to obtain a cargo that contains only one Cys residue for coupling to HTRA1.

Third, the cargo already contains or is engineered to contain a binding site for HTRA1's PDZ domain. The binding site of HTRA1's PDZ domain typically consists of four C-terminal residues. This mechanism of interaction is non-covalent, enabling to establish conditions where the cargo can detach and diffuse away from HTRA1 e.g. to reach its final cellular localisation. The PDZ domain of the carrier, i.e. HTRA1, could be replaced by another PDZ domain, a potential feature of which could be increased affinity to a ligand (in this case the C-terminus of the cargo).

In specific embodiments, the present invention provides an in vitro method for transport of a molecule of interest into a cell, comprising the steps of
(a) providing a conjugate of the molecule of interest and a HTRA1 polypeptide;
(b) contacting the cell with the conjugate in the absence of any other means suitable for inducing uptake of polypeptides into a cell;
(c) incubating the cell in the presence of the conjugate for a period of time sufficient for uptake of the conjugate into the cell;
wherein the HTRA1 polypeptide comprises the HTRA1 protease domain or an amino acid sequence derived therefrom which shares an identity with the amino acid sequence of the HTRA1 protease domain over its entire length of at least 80%.

The HTRA1 polypeptide used in the method for transport of a molecule of interest into a cell may be any HTRA1 polypeptide having the ability to facilitate uptake into a cell. Especially, the HTRA1 polypeptide comprises the protease domain of HTRA1 or an amino acid sequence derived therefrom. In particular, the HTRA1 polypeptide may comprise the amino acid sequence according to amino acid positions 158 to 373 of SEQ ID NO: 1, 2 or 3, in particular of SEQ ID NO: 1, or an amino acid sequence derived therefrom.

In certain embodiments, the HTRA1 polypeptide may comprises the protease domain and the PDZ domain of HTRA1 or an amino acid sequence derived therefrom, in particular the amino acid sequence according to amino acid positions 158 to 480 of SEQ ID NO: 1, 2 or 3, in particular of SEQ ID NO: 1, or an amino acid sequence derived therefrom. In further embodiments, the HTRA1 polypeptide may comprises the entire HTRA1, in particular full length mature HTRA1, or an amino acid sequence derived therefrom, especially the amino acid sequence according to amino acid positions 23 to 480 of SEQ ID NO: 1, 2 or 3, in particular of SEQ ID NO: 1, or an amino acid sequence derived therefrom.

To minimise side effects of the biological activity of the delivery agent itself, HTRA1 can be modified to lose all of its proteolytic activity and its binding sites for interaction partners. The HTRA1 polypeptide may have protease activity or not and in particular does not have a protease activity. In specific embodiments, the HTRA1 polypeptide does not have any protease activity or has a reduced protease activity in comparison to wildtype HTRA1. In particular, in the HTRA1 polypeptide the amino acid residue corresponding to serine at position 328 of SEQ ID NO: 1 is any amino acid residue except serine, such as for example alanine.

In certain embodiments, the HTRA1 polypeptide is mutated so as to reduce its substrate binding affinity. There are two binding sites for substrates in HTRA1, the active site and the PDZ domain. In certain embodiments, the HTRA1 polypeptide does not contain a PDZ domain. This embodiment in particular is combined with the embodiments wherein the HTRA1 polypeptide is covalently coupled to the molecule of interest. In a further embodiment, the substrate binding site in the protease domain is mutated to loose substrate binding pockets. In specific embodiments, loop L1 corresponding to amino acid positions 325 to 327 of SEQ ID NO: 1 and/or loop L2 corresponding to amino acid positions 346 to 350 of SEQ ID NO: 1 and or loop LD corresponding to amino acid positions 284 to 289 of SEQ ID NO: 1 are partially or entirely deleted and/or mutated in the HTRA1 polypeptide. In another embodiment, the substrate binding site is occupied by modified peptides including but not limited to peptidic boronic acids or chloromethylketones. Examples of peptidic boronic acids or chloromethylketones are DPMFKLboroV, DPMFKLV-cmk, VFNTLPMMGKASPboroV and VFNTLPMMGKASPV-cmk, wherein "boro" is a boronic acid and "cmk" is a chloromethylketone. In an even further embodiment, the HTRA1 polypeptide is modified to comprise a HTRA1 protease domain variant containing an additional peptide loop that interacts with but is not cleaved by the active site of the protease domain. The additional peptide loop may comprise or consist of the amino acid sequence according to SEQ ID NO: 6.

Native HTRA1 is a homooligomeric protein. However, certain mutations can cause HTRA1 to become monomeric. Monomeric HTRA1 could be a preferred carrier if e.g. the cargo will interact with other proteins e.g. when part of a multiprotein complex because complex association of the internalized cargo might be hindered if the carrier is too large. Therefore, in specific embodiments, the HTRA1 polypeptide is mutated so as to reduce or remove its ability to oligomerize. In particular, the HTRA1 polypeptide is a monomer. In these embodiments, the HTRA1 polypeptide preferably comprises one or more amino acid substitutions compared to the amino acid sequence according to any one of SEQ ID NOs: 1 to 3, including but are not limited to Arg166His, Ala173Thr, Arg274Gln and Gly295Arg.

In specific embodiments, the HTRA1 polypeptide comprises a cellular localization signal. Suitable examples of such cellular localization signals are nuclear, peroxisomal or mitochondrial localization sequences. Exemplary cellular localization signals are selected from the group consisting of the nuclear localization signal having the amino acid sequence of SEQ ID NO: 7, the C terminal peroxisomal localization signal having the amino acid sequence of -Ser-Lys-Leu-COOH, the N terminal peroxisomal localization signal having the amino acid sequence of SEQ ID NO: 8, and the mitochondrial import signal having the amino acid sequence of SEQ ID NO: 9.

The molecule of interest may be any molecule including in particular proteins, polypeptides, nucleic acids, organic compounds, radionuclides and therapeutically active substances. The molecule of interest also includes complexes of two or more separate molecules. In particular, the molecule of interest is a polypeptide.

The conjugate provided in step (a) and contacted with the cell in step (b) may comprise or consist of a conjugate wherein the molecule of interest and the HTRA1 polypeptide are covalently conjugated to each other or comprise or consist of the molecule of interest and the HTRA1 polypeptide as separate compounds which are non-covalently bound to each other. Furthermore, the conjugate may comprise additional components, for example components improving the stability and/or solubility of the conjugate.

The molecule of interest and the HTRA1 polypeptide may by covalently conjugated to each other or non-covalently bound to each other in order to form the conjugate. In embodiments wherein the molecule of interest is a polypeptide, covalent conjugation may for example include embodiments wherein the molecule of interest and the HTRA1 polypeptide are fused together and form a hybrid polypeptide, or conjugated via a disulfide bond.

The molecule of interest being a polypeptide may be fused to the HTRA1 polypeptide either directly or via a linker. A direct fusion refers to hybrid polypeptides wherein the sequence of the molecule of interest directly follows or precedes the sequence of the HTRA1 polypeptide without any intermediate amino acids between these two sequences. A fusion via a linker refers to hybrid polypeptides wherein one or more amino acids are present between the sequence of the molecule of interest and the sequence of the HTRA1 polypeptide. These one or more amino acids form the linker between the molecule of interest and the HTRA1 polypeptide.

The linker may in principle have any number of amino acids and any amino acid sequence which are suitable for linking the molecule of interest and the s HTRA1 polypeptide. In certain embodiments, the linker between the molecule of interest and the HTRA1 polypeptide comprises at least 3, preferably at least 5 or at least 10 amino acids. In specific embodiments, the linker comprises a protease cleavage site, in particular for an intracellular protease.

In further embodiments, the molecule of interest being a polypeptide may covalently conjugated to the HTRA1 polypeptide via a disulfide bond. In these embodiments, the HTRA1 polypeptide contains a cysteine residue and the molecule of interest contains a cysteine residue. In particular, a cysteine residue may be attached to the N terminus or C terminus of the HTRA1 polypeptide. The disulfide bond may be broken by the reducing environment of the cytosol after uptake of the conjugate.

In further embodiments, the molecule of interest being a polypeptide and the HTRA1 polypeptide are non-covalently bound to each other. In these embodiments, the HTRA1 polypeptide preferably comprises a PDZ domain and the molecule of interest comprises a PDZ binding peptide. For example, the HTRA1 polypeptide may comprise the PDZ domain of HTRA1, in particular a PDZ domain having the amino acid sequence according to amino acid positions 365 to 467 of any one of SEQ ID NOs: 1 to 3, such as SEQ ID NO: 1, or an amino acid sequence derived therefrom. In another embodiment, the HTRA1 polypeptide may comprise any other PDZ domain known in the art. The molecule of interest comprises in these embodiments a PDZ binding peptide which is able to bind to the PDZ domain present in the HTRA1 polypeptide. The PDZ binding peptide in particular specifically binds to the PDZ domain. In embodiments wherein the HTRA1 polypeptide comprises the PDZ domain of HTRA1, the molecule of interest preferably comprises a PDZ binding peptide comprising the amino acid sequence according to SEQ ID NO: 10. Further examples of PDZ binding peptides comprising an amino acid sequence selected from the group consisting of SEQ ID NOs: 11 to 16. The PDZ binding peptide preferably is present at the C terminus of the molecule of interest.

The molecule of interest and/or the HTRA1 polypeptide may comprise a cellular localization signal. Suitable examples are nuclear localization sequences, peroxisomal localization sequences and mitochondrial localization sequences. Exemplary cellular localization signals are selected from the group consisting of the nuclear localization signal having the amino acid sequence of SEQ ID NO: 7, the C terminal peroxisomal localization signal having the amino acid sequence -Ser-Lys-Leu-COOH, the N terminal peroxisomal localization signal having the amino acid sequence of SEQ ID NO: 8, and the mitochondrial import signal having the amino acid sequence of SEQ ID NO: 9.

The contacting step (b) and the incubation step (c) of the method for transport of a molecule of interest into a cell are in particular performed in the absence of any other means suitable for inducing uptake of polypeptides into a cell. Such other means suitable for inducing uptake of polypeptides into a cell include cell penetrating peptides, nanoparticles, lipophilic transduction reagents.

The method for transport of a molecule of interest into a cell is not performed in the human body and in particular not performed in the human or animal body. The method for transport of a molecule of interest into a cell is an in vitro method.

The cell is contacted with the conjugate for example by adding the conjugate to the cell environment, for example into the cell medium. In certain embodiments, the cell is contacted with the conjugate in a concentration of at least 1 µg/ml. Specifically, the conjugate is used in a concentration of at least 5 µg/ml, at least 10 µg/ml, at least 20 µg/ml, at least 50 µg/ml, or at least 100 µg/ml. The conjugate may in particular be used in a concentration in the range of from 1 µg/ml to 1000 µg/ml, especially from 10 µg/ml to 150 µg/ml. In further embodiments, the cell is contacted with the conjugate in a concentration of at least 0.05 µM. Specifically, the conjugate is used in a concentration of at least 0.2 µM, at least 0.5 µM, at least 1 µM, at least 2 µM, or at least 5 µM. The conjugate may in particular be used in a concentration in the range of from 0.05 µM to 50 µM, especially from 0.5 µM to 5 µM. In specific embodiments, the concentration of the conjugate as specified above refers to the concentration of the HTRA1 polypeptide as part of the conjugate. The molecule of interest and any further potential components of the conjugate are not taken into account in these embodiments.

In the third step, the cell is incubated in the presence of the conjugate. The time of incubation is chosen so as to be sufficient for uptake into the cells of at least a part of the conjugates contacted with the cells. In particular, the amount of conjugates taken up by the cells after the incubation step should be sufficient to achieve the desired amount of uptake of the molecule of interest into the cells. In certain embodiments, the cell is incubated in the presence of the conjugate for at least 1 min, in particular at least 5 min, at least 10 min, at least 30 min or at least 60 min. The cell may in particular be incubated in the presence of the conjugate for a period of time in the range of from 1 min to 120 min, especially 10 min to 60 min.

Also described is a method for transport of a molecule of interest into a cell, comprising the steps of
(a) providing a conjugate of the molecule of interest and a HTRA3 polypeptide;
(b) contacting the cell with the conjugate in the absence of any other means suitable for inducing uptake of polypeptides into a cell;
(c) incubating the cell in the presence of the conjugate for a period of time sufficient for uptake of the conjugate into the cell.

Futher described is a method for transport of a molecule of interest into a cell, comprising the steps of
(a) providing a conjugate of the molecule of interest and a HTRA4 polypeptide;
(b) contacting the cell with the conjugate in the absence of any other means suitable for inducing uptake of polypeptides into a cell;
(c) incubating the cell in the presence of the conjugate for a period of time sufficient for uptake of the conjugate into the cell.

The features and embodiments described above with respect to the method for transport of a molecule of interest into a cell using a HTRA1 polypeptide likewise apply to the method for transport of a molecule of interest into a cell using a HTRA3 polypeptide or a HTRA4 polypeptide.

In embodiments where the HTRA3 polypeptide comprises the HTRA3 protease domain or an amino acid sequence derived therefrom, the HTRA3 polypeptide in particular comprises the amino acid sequence according to amino acid positions 132 to 350 of SEQ ID NO: 4, or an amino acid sequence derived therefrom. In certain embodiments, the HTRA3 polypeptide may comprises the protease domain and the PDZ domain of HTRA3 or an amino acid sequence derived therefrom, in particular the amino acid sequence according to amino acid positions 132 to 453 of SEQ ID NO: 4 or an amino acid sequence derived therefrom. In further embodiments, the HTRA3 polypeptide may comprises the entire HTRA3, in particular full length mature HTRA3, or an amino acid sequence derived therefrom, especially the amino acid sequence according to amino acid positions 18 to 453 of SEQ ID NO: 4, or an amino acid sequence derived therefrom.

In embodiments where the HTRA3 polypeptide does not have any protease activity or has a reduced protease activity in comparison to wildtype HTRA3, the amino acid residue corresponding to serine at position 305 of SEQ ID NO: 4 in particular is any amino acid residue except serine, such as for example alanine, in the HTRA3 polypeptide.

In embodiments where the HTRA3 polypeptide is mutated so as to reduce or remove its ability to oligomerize, the HTRA3 polypeptide preferably comprises one or more amino acid substitutions compared to the amino acid sequence according to SEQ ID NO: 4, including but are not limited to Arg136His, Ala144Thr, Arg251Gln and Gly272Arg.

In embodiments where the HTRA3 polypeptide is mutated so as to reduce its substrate binding affinity, the substrate binding site in the protease domain may be mutated to loose substrate binding pockets. In particular, loop L1 corresponding to amino acid positions 300 to 304 of SEQ ID NO: 4 and/or loop L2 corresponding to amino acid positions 323 to 327 of SEQ ID NO: 4 and or loop LD corresponding to amino acid positions 261 to 267 of SEQ ID NO: 4 are partially or entirely deleted and/or mutated in the HTRA3 polypeptide.

In embodiments where the HTRA4 polypeptide comprises the HTRA4 protease domain or an amino acid sequence derived therefrom, the HTRA4 polypeptide in particular comprises the amino acid sequence according to amino acid positions 156 to 371 of SEQ ID NO: 5, or an amino acid sequence derived therefrom. In certain embodiments, the HTRA4 polypeptide may comprises the protease domain and the PDZ domain of HTRA4 or an amino acid sequence derived therefrom, in particular the amino acid sequence according to amino acid positions 156 to 476 of SEQ ID NO: 5 or an amino acid sequence derived therefrom. In further embodiments, the HTRA4 polypeptide may comprises the entire HTRA4, in particular full length mature HTRA4, or an amino acid sequence derived therefrom, especially the amino acid sequence according to amino acid positions 32 to 476 of SEQ ID NO: 5, or an amino acid sequence derived therefrom.

In embodiments where the HTRA4 polypeptide does not have any protease activity or has a reduced protease activity in comparison to wildtype HTRA4, the amino acid residue corresponding to serine at position 326 of SEQ ID NO: 5 in particular is any amino acid residue except serine, such as for example alanine, in the HTRA4 polypeptide.

In embodiments where the HTRA4 polypeptide is mutated so as to reduce or remove its ability to oligomerize, the HTRA4 polypeptide preferably comprises one or more amino acid substitutions compared to the amino acid sequence according to SEQ ID NO: 5, including but are not limited to Arg164His, Ala171Thr, Arg272Gln and Gly293Arg.

In embodiments where the HTRA4 polypeptide is mutated so as to reduce its substrate binding affinity, the substrate binding site in the protease domain may be mutated to loose substrate binding pockets. In particular, loop L1 corresponding to amino acid positions 323 to 325 of SEQ ID NO: 5 and/or loop L2 corresponding to amino acid positions 344 to 349 of SEQ ID NO: 5 and or loop LD corresponding to amino acid positions 281 to 287 of SEQ ID NO: 5 are partially or entirely deleted and/or mutated in the HTRA4 polypeptide.

### The conjugate comprising a therapeutic agent and a HTRA polypeptide

The present application also describes a conjugate comprising a therapeutic agent and a HTRA1, HTRA3 or HTRA4 polypeptide. The present invention pertains to a conjugate comprises a therapeutic agent and a HTRA1 polypeptide, wherein the HTRA1 polypeptide comprises the HTRA1 protease domain or an amino acid sequence derived therefrom which shares an identity with the amino acid sequence of the HTRA1 protease domain over its entire length of at least 80%.

In certain embodiments, the conjugate is a conjugate as described herein with respect to the method for transport of a molecule of interest into a cell is used, wherein the molecule of interest is the therapeutic agent. In particular, a HTRA1 polypeptide as described herein with respect to the method for transport of a molecule of interest into a cell is used. In specific embodiments, the HTRA1 polypeptide comprises and in particular consists only of the HTRA1 protease domain and optionally the HTRA1 PDZ domain. In one embodiment, the conjugate does not comprise the full-length HTRA1 with or without the localization signal peptide. In other embodiments, the HTRA3 polypeptide comprises and in particular consists only of the HTRA3 protease domain and optionally the HTRA3 PDZ domain. In further embodiments, the HTRA4 polypeptide comprises and in particular consists only of the HTRA4 protease domain and optionally the HTRA4 PDZ domain.

The therapeutic agent present in the conjugate may be any therapeutic agent which is therapeutically effective intracellularly, i.e. takes effect inside of a cell, in particular a cell involved in a disease. The therapeutic agent in particular is a therapeutic agent as described herein with respect to the therapeutic use of a conjugate comprising a HTRA1, HTRA3 or HTRA4 polypeptide.

The conjugate preferably comprises the therapeutic agent covalently coupled to the HTRA1, HTRA3 or HTRA4 polypeptide, either directly or via a linker.

### The therapeutic use of a conjugate comprising a HTRA polypeptide

The conjugates comprising a molecule of interest and a HTRA1, HTRA3 or HTRA4 polypeptide as described herein furthermore can be used in therapeutic applications. In particular, the HTRA1, HTRA3 or HTRA4 polypeptide can be used to deliver therapeutic agents into cells in order to treat a disease.

Hence, in a fifth aspect the present invention is directed to a conjugate comprising a therapeutic agent and a HTRA1 polypeptide for use in the treatment of a disease, wherein a pharmaceutical preparation containing the conjugate is locally applied to the site of the disease, and wherein the HTRA1 polypeptide comprises the HTRA1 protease domain or an amino acid sequence derived therefrom which shares an identity with the amino acid sequence of the HTRA1 protease domain over its entire length of at least 80%. The presence or increased amount of the therapeutic agent in the cells at the site of the disease is beneficial for treating the disease.

In certain embodiments, a conjugate as described herein with respect to the method for transport of a molecule of interest into a cell is used, wherein the molecule of interest is the therapeutic agent, or as described herein with respect to the conjugate comprising a therapeutic agent and a HTRA1 polypeptide. In particular, a HTRA1, polypeptide as described herein with respect to the method for transport of a molecule of interest into a cell is used.

The therapeutic agent for example may be a chemotherapeutic and/or cytotoxic agent or a radionuclide. Specific examples of chemotherapeutic agents that can be used as therapeutic agent include alkylating agents such as cisplatin, anti-metabolites, plant alkaloids and terpenoids, vinca alkaloids, podophyllotoxin, taxanes such as taxol, topoisomerase inhibitors such as irinotecan and topotecan, or antineoplastics such as doxorubicin. In these embodiments, the disease preferably is cancer and the pharmaceutical composition is locally applied to the tumor site.

A classical exemplary indication is cancer where tumour suppressors are lost by genetic alterations. The restoration of tumour suppressor levels in cancer cells via HTRA1/3/4-mediated internalisation will drive cancer cells into apoptosis. Therefore, in specific embodiments the disease to be treated is cancer and the therapeutic agent is a tumour suppressor. Exemplary tumour suppressors are TP53, PTEN, APC, CD95, ST5, YPEL3, ST7, and ST14. In these embodiments, the pharmaceutical composition is locally applied to the tumor site.

Furthermore, the treatment may be an enzyme replacement therapy. In these embodiments, the therapeutic agent is the enzyme which is to be replaced, i.e. the enzyme which activity is lacking or low in the patient. The pharmaceutical composition may be applied to sites where the activity of the enzyme is needed or may be administered systemically.

In specific embodiments, the pharmaceutical preparation does not contain any other means suitable for inducing uptake of polypeptides into a cell. The conjugate is locally applied, especially to the site of the disease, in order to transport the therapeutic agent into the affected cells. The conjugate may be applied topically, by injection or by eye drops, depending on the site of the disease.

The present application further describes a method for treatment of a disease, comprising administering a conjugate of a therapeutic agent and a HTRA1, HTRA3 or HTRA4 polypeptide to a patient in need thereof. This treatment includes locally applying a pharmaceutical preparation containing the conjugate to the site of the disease. The patient benefits from transferring the therapeutic agent into cells at the site of the disease. In particular, the pharmaceutical preparation does not contain any other means suitable for inducing uptake of polypeptides into a cell. The features and embodiments described above with respect to the HTRA1, HTRA3 or HTRA4 polypeptide for use in the treatment of a disease also apply likewise to the method for treatment of a disease.

Numeric ranges described herein are inclusive of the numbers defining the range. The headings provided herein are not limitations of the various aspects or embodiments of this invention which can be read by reference to the specification as a whole. According to one embodiment, subject matter described herein as comprising certain steps in the case of methods or as comprising certain ingredients in the case of compositions refers to subject-matter consisting of the respective steps or ingredients. It is preferred to select and combine preferred aspects and embodiments described herein and the specific subject-matter arising from a respective combination of preferred embodiments also belongs to the present disclosure.

### FIGURES

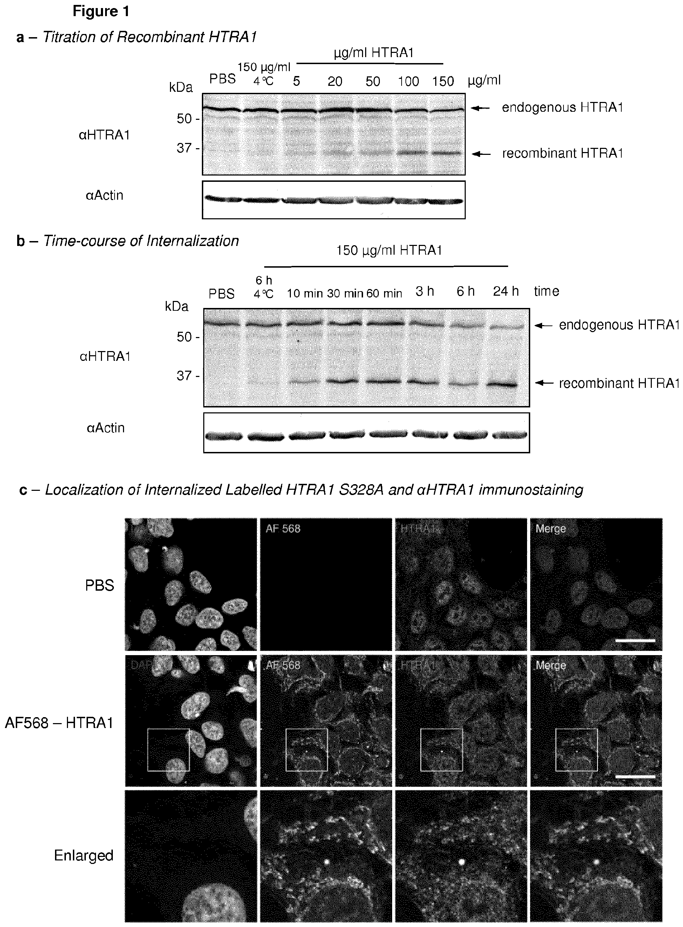

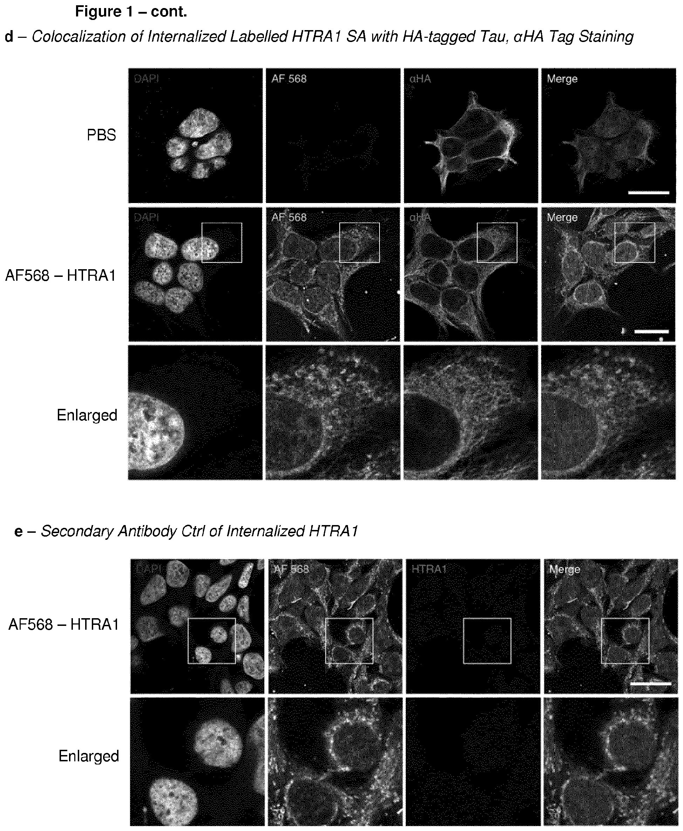

**Fig. 1** shows internalisation of recombinant HTRA1 by cultured 293T HEK cells. Cells were incubated for 6 h with culture medium conditioned with increasing concentrations of HTRA1 a) or with a fixed amount of HTRA1 (150 µg/ml = 5.54 µM) for the incubation times indicated (b). Lysates of trypsinised cells were subjected to SDS-PAGE and immunoblotting using antibodies against HTRA1 and Actin (loading control). As controls, cells were treated with PBS or 5.54 µM HTRA1 followed by incubation at 4°C for 6 h. (c-d) Uptake of fluorescently labelled HTRA1 S328A by 293T HEK cells followed by confocal microscopy. Cells were treated with 50 µg/ml (= 1.85 µM) HTRA1 or PBS (control) for 16 h, followed by methanol fixation and immunostaining against HTRA1 (c) or the HA tag (d) of transiently overexpressed tau as indicated. DAPI was used as nuclear counterstain. The images show the dispersed cytoplasmic or vesicular localisation of internalised HTRA1 S328A (labelled with AF 568) (c) and positive immunostaining of HTRA1 and colocalisation with its native cytoplasmic substrate tau (αHA antibody) (d). (e) Secondary antibody (2°AB) control of internalised, fluorescently labelled HTRA1. 293T HEK cells were treated with HTRA1 conditioned medium for 20 h, followed by fixation and staining as described above except for the primary antibody against HTRA1 being omitted in order to exclude unspecific staining of labelled HTRA1 or the AlexaFluor 568 dye by the secondary antibody. Scale bars, 25 µm.

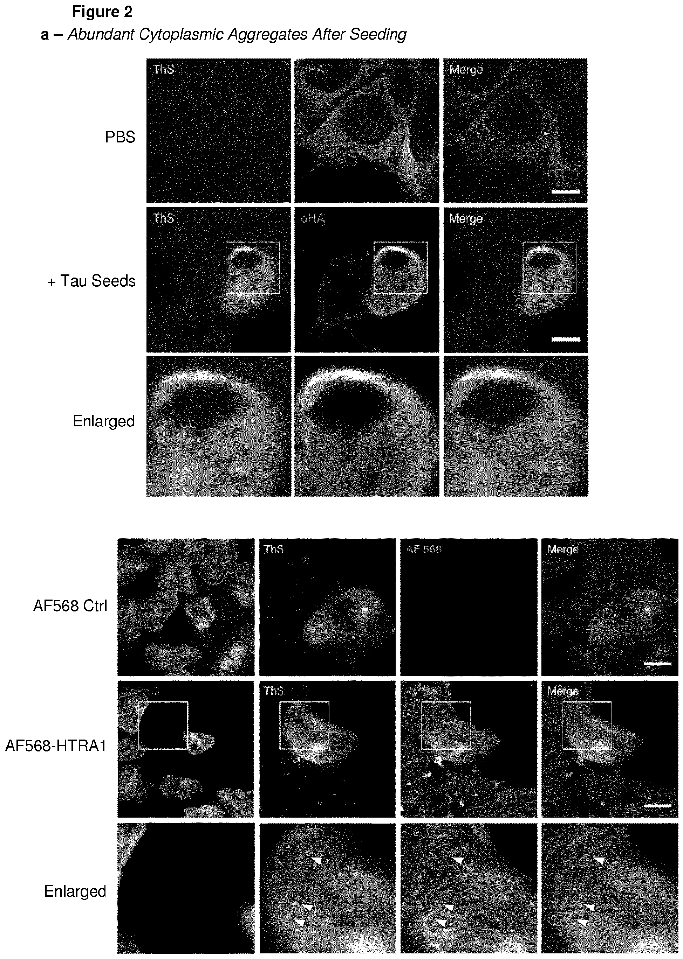

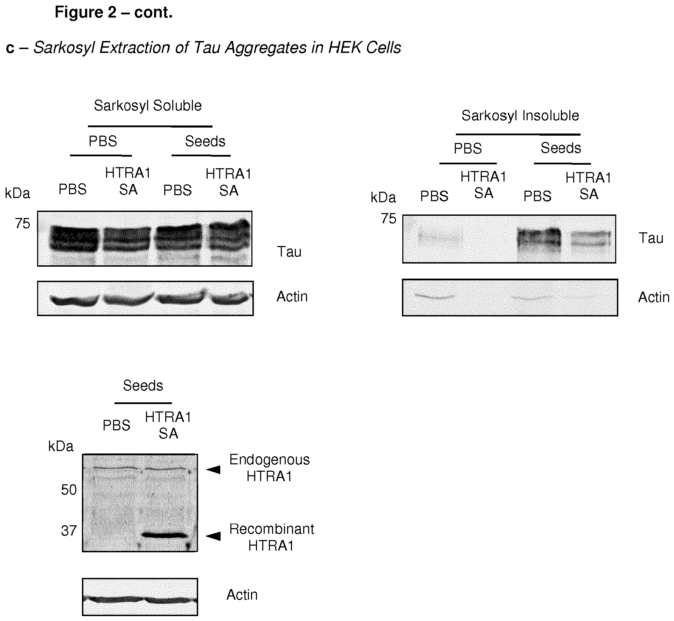

**Fig. 2** shows a cell culture model of tau aggregation and sarkosyl extraction of HA-tagged tau. (a) Aggregation of cytoplasmic tau was induced by seeding with MTBD fibrils in 293T HEK cells transiently overexpressing HA-tagged P301L tau. Fixed cells were stained with the amyloid specific fluorescent dye Thioflavin S (ThS, green) and immunostained against the HA tag of overexpressed tau (αHA, red). (b) Cells containing seeded tau filaments were treated with AlexaFluor 568 labelled HTRA1 S328A (1.85 µM) or the dye alone (both shown in red) followed by ThS staining (green) and ToPro3 nuclear counterstaining (blue). Arrowheads indicate regions of distinct colocalisation of labelled HTRA1 and tau aggregates. Scale bars, 10 µm. (c) Cells treated with PBS or seeded with MTBD fibrils were subsequently treated with recombinant HTRA1 S328A (5.54 µM) or PBS for 20 h. Lysates were sarkosyl extracted to assess the amounts of soluble (left panel) versus aggregated (right panel) tau using α tau antibodies. Sarkosyl soluble fractions were also immunoblotted against HTRA1 to show internalisation of recombinant HTRA1. Actin levels are loading controls or show that sarkosyl pellets did not contain cytoplasmic proteins (right panel). Note that recombinant HTRA1 migrates at 37 kDa (i.e. below the native HTRA1 of 51 kDa) because it is lacking the N-terminus for which no function has been detected so far (Eigenbrot et al., 2012). Representative data of three independent experiments are shown.

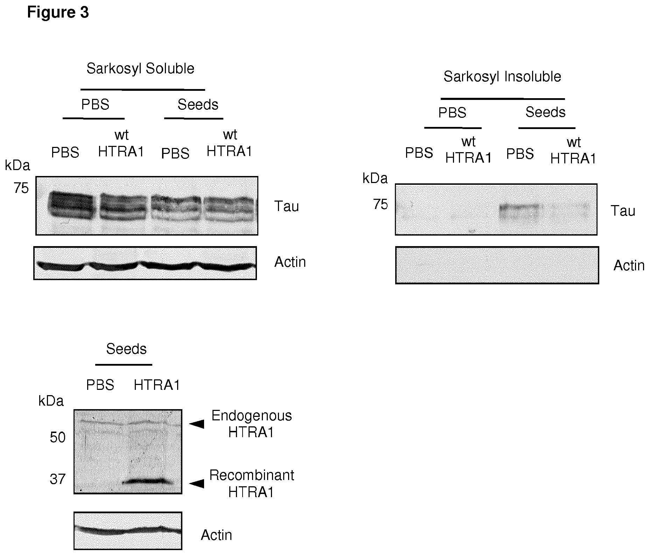

**Fig. 3** shows enhanced proteolysis of tau aggregates by internalised HTRA1 in a cellular model of tau aggregation. 293T HEK cells were treated as described in Fig. 2. After seeding, cells were treated with culture medium conditioned with proteolytically active HTRA1 (wt) (5.54 µM) or PBS for 20 h followed by lysis, sarkosyl extraction and immunoblotting of sarkosyl soluble (left panel) and insoluble (pellet, right panel) fractions against tau, and immunoblotting of the sarkosyl soluble fractions against HTRA1 to detect the uptake of recombinant wt HTRA1 (lower panel). Representative data of three independent experiments are shown.

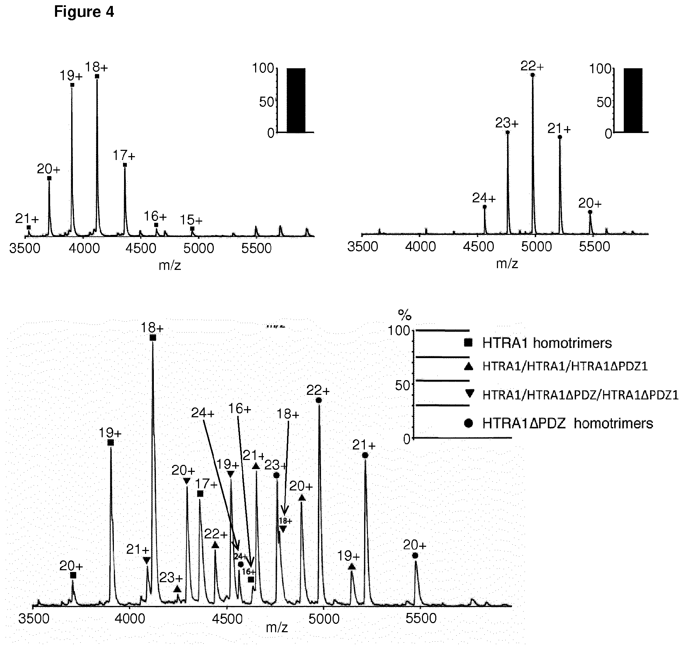

Fig. 4 shows monomer exchange in mixed HTRA1 variants. Purified HTRA1 was incubated for 30 min with equal amounts of purified HTRA1ΔPDZ. Subsequently, these samples were analysed by native mass spectrometry. The obtained spectra are shown. HTRA1 trimers are marked by signs: HTRA1 homotrimers (rectangle), HTRA1ΔPDZ homotrimers (dot), HTRA1/HTRA1/HTRA1ΔPDZ1 (triangle), HTRA1/HTRA1ΔPDZ1/HTRA1ΔPDZ1 (inverted triangle). The percentage of each trimer is highlighted in the insets. The charge states are indicated above the peaks.

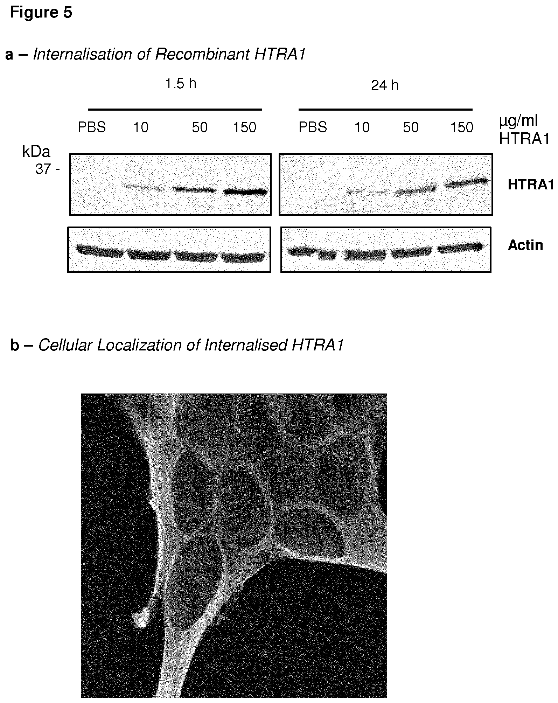

**Fig. 5** shows internalisation of recombinant HTRA1 S328A by cultured SHSY5Y cells. a) Cells were incubated for the time points indicated with culture medium conditioned with increasing concentrations of HTRA1 S328A. Lysates of trypsinised cells were subjected to SDS-PAGE and immunoblotting using antibodies against HTRA1 and Actin (loading control). As controls, cells were treated with PBS instead of HTRA1. b) Cellular localisation of internalised recombinant HTRA1 S328A labeled with DyLight 488 in cultured SHSY5Y cells followed by confocal laser scanning microscopy using a Leica SP5 microscope.

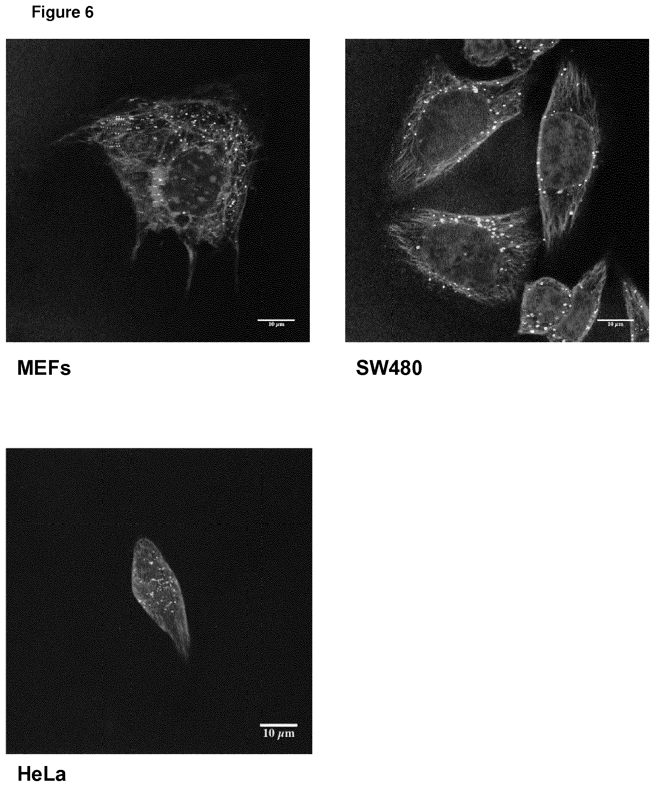

**Fig. 6** shows the cellular localisation of internalised recombinant HTRA1 in cultured mouse embryonal fibroblasts (MEF) as well as in SW480 and HeLa cells. Cells were incubated with HTRA1 S328A labeled with DyLight 488 for 6 h at 37°C. Subsequently, cells were washed, fixed and permeabilised. α-tubulin was stained with a mouse anti-α-Tubulin antibody and nuclei were stained with DAPI. Images were obtained at a Leica SP5 confocal laser scanning microscope.

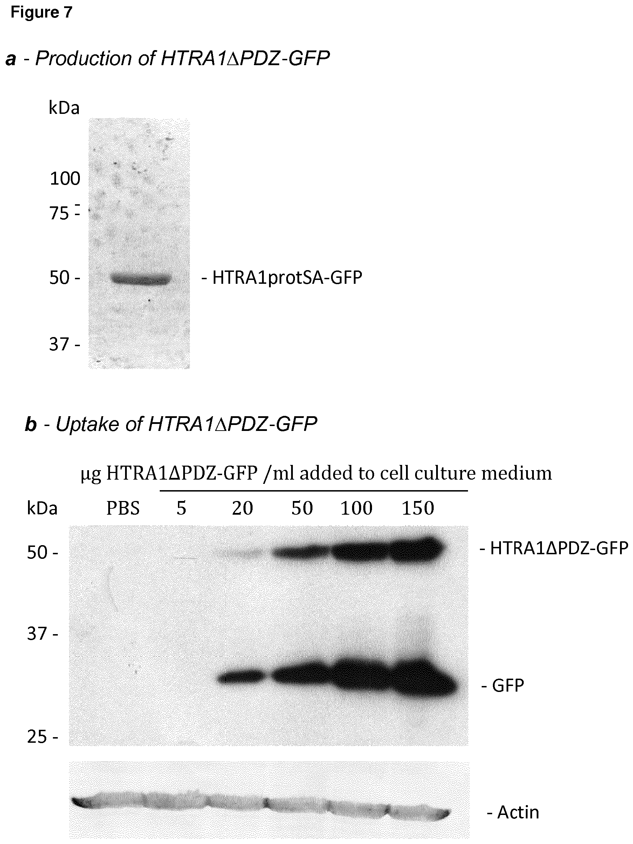

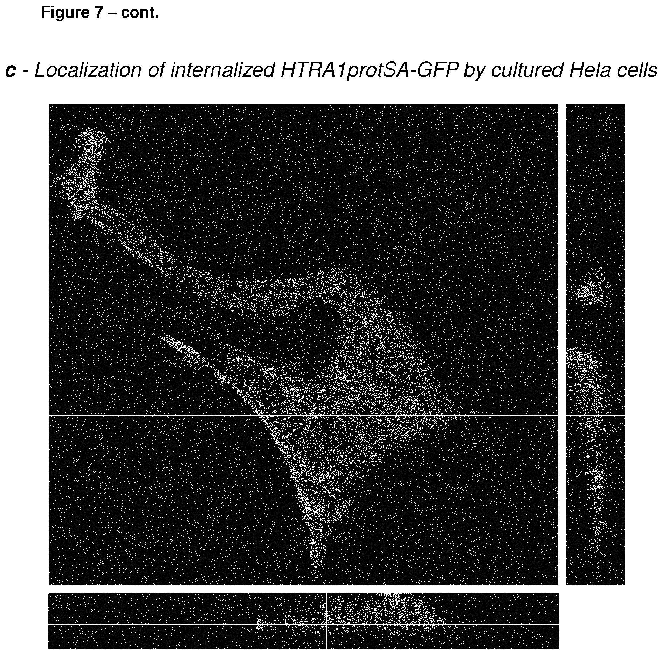

**Fig. 7** shows uptake of cargo coupled to HTRA1. a) *Purified HTRA1*Δ*PDZ-GFP hybrid protein is fluorescent.* Purified HTRA1ΔPDZ-GFP hybrid protein was analysed via SDS-PAGE. The fluorescence of GFP was visualised by a Typhoon reader at 488 nm. The detected protein migrated near 50 kDa i.e. at the expected molecular mass of the HTRA1ΔPDZ-GFP hybrid protein. b) *Uptake of HTRA1*Δ*PDZ-GFP by SW480 cells.* SW480 cells were incubated with various amounts of HTRA1ΔPDZ-GFP in serum free RPMI medium. PBS was added as a control. After 24 h cells were harvested by trypsinisation (to remove extracellular HTRA1ΔPDZ-GFP) and lysed. Whole cells extracts were subjected to SDS-PAGE followed by Western bloting using an anti-GFP antibody. Actin served as an internal loading control. c) Localisation of internalised HTRA1ΔPDZSA-GFP by cultured HeLa cells. Cells were incubated in serum free DMEM medium containing 50 µg/ml HTRA1ΔPDZSA-GFP for 6 h followed by paraformaldehyde fixation. DAPI was used as nuclear counterstain, Phalloidin to stain the Actin-cytoskeleton. The image shows the dispersed cytoplasmic localization of internalised HTRA1ΔPDZSA-GFP. Orthogonal views of the XZ- and YZ-axis highlight the internal localization.

### EXAMPLES

### Example 1: Internalization of recombinant HTRA1 by cultured mammalian cells.

During studies addressing the potential physiological relevance of a disaggregase activity of HTRA1 towards protein fibrils that are hallmarks of many protein folding diseases, we established a cell based assay involving disaggregation and digestion of intracellular tau fibrils by HTRA1. To reduce the complexity of the cell based assay including the problem arising from heterogeneity and uncontrolled protein levels within a batch of cells transfected with plasmids, an alternative approach of introducing defined amounts of HTRA1 into human cells was required. We therefore tested whether human cells would take up purified recombinant HTRA1 without using protein transfection reagents. To test this hypothesis, 293T HEK cells were treated with culture medium conditioned with HTRA1 in concentrations between 5 and 150 µg/ml followed by incubation for 6 h (Fig. 1a) or with 150 µg/ml HTRA1 for various periods of time (Fig. 1b). Subsequently, lysates of trypsinised cells (extracellular HTRA1 is highly sensitive to trypsin) were subjected to SDS-PAGE and Western blots using antibodies against HTRA1 and actin (loading control). Control cells were treated with PBS (no HTRA1) or 150 µg/ml HTRA1 followed by incubation at 4°C instead of 37°C for 6 h (Fig. 1a,b). Incubation at 4°C abrogates active uptake processes such as endocytosis but does not inhibit passive internalization as observed e.g. for cell penetrating peptides (Ter-Avetisyan et al., 2009). The Western blots revealed that recombinant HTRA1 was indeed internalised from the medium. Moreover, these levels correspond roughly to the levels of endogenous HTRA1 and the introduced HTRA1 was detectable for at least 24 h following uptake. Note that recombinant HTRA1 migrates at 37 kDa (i.e. below the native HTRA1 of 51 kDa) because it is lacking the N-terminus for which no function has been detected so far (Eigenbrot et al., 2012).

To address the cellular localisation of internalised HTRA1, fluorescently labelled HTRA1 S328A was subjected to confocal microscopy. Cells were treated with 50 µg/ml (1.85 µM) HTRA1 or PBS for 16 h, followed by immunostaining using antibodies against HTRA1 (Fig. 1c) These data show that internalised HTRA1 is cytosolic, equally distributed among cells, and that the alexa dye AF568 is not detachted from HTRA1 during or following its uptake into cells (Fig 1a-c).

In addition, internalised HTRA1 should colocalise with endogenous tau. Therefore, localisation of fluorescently labelled HTRA1 S328A with the HA tag of transiently overexpressed tau was investigated (Fig. 1d). The images show colocalization of internalized HTRA1 S328A with its native cytosolic substrate tau, as well as the dispersed cytoplasmic or vesicular localisation of endogenous HTRA1 and internalised HTRA1 S328A (Fig. 1c,d). The secondary antibody control shows no HTRA1 staining (Fig. 1e)

### Example 2: Disaggregation of intracellular tau fibrils.

Aggregation of cytoplasmic tau was induced by transfection of seeds of MTBD fibrils into 293T HEK cells transiently overexpressing HA-tagged tau. Labelling of cells with the amyloid specific fluorescent dye Thioflavin S (ThS, green) and immunostained overexpressed tau (αHA, red) show that cells are loaded with large amounts of aggregated tau protein (Fig. 2a). In addition, cells treated with 1.85 µM recombinant labelled HTRA1 S328A (or as a control just with the dye AlexaFluor 568 but no HTRA1) followed by ThS staining (green) and ToPro3 nuclear counterstaining (blue), reveal distinct colocalization of labelled HTRA1 and tau aggregates (Fig. 2b, see arrowheads highlighting exemplary regions).

Having established a cell culture model for tau aggregation, we tested the effect of HTRA1 S328A on the levels of aggregated tau in cells. Cells that transiently overexpressed HA tagged tau were transfected with seeds of aggregated MTBD tau or treated with PBS alone (control). Subsequently, these cells were treated with 1.85 µM recombinant HTRA1 S328A or treated with PBS alone (control) and were incubated for 20 h, followed by N-lauroylsarcosine (sarkosyl) extraction of cell lysates. Soluble fractions from this extraction contain soluble tau whereas the pellet fractions contain the tau aggregates. These fractions were subjected to Western blotting using antibodies against tau and HTRA1. Notably, HTRA1 S328A treated cells had less tau fibrils compared to the PBS controls, while there were no marked differences of soluble tau levels (Fig. 2c).

### Example 3: Proteolysis of intracellular tau fibrils.

Using the cell culture model for tau aggregation established above, we asked whether internalised proteolytically active HTRA1 would reduce the levels of aggregated tau in cells. Therefore, cells that transiently overexpressed HA tagged tau were transfected with seeds of aggregated MTBD tau or treated with PBS alone (control). Subsequently, these cells were treated with 5.54 µM recombinant HTRA1 or with PBS alone (control) and were incubated for 20 h. Subsequently, sarkosyl extraction of cell lysates was carried out. Soluble fractions from this extraction contain soluble tau whereas the pellet fractions contain the tau aggregates. These fractions were subjected to Western blotting using antibodies against tau and HTRA1. As expected, cells treated with proteolytically active HTRA1 had less tau fibrils compared to the PBS controls (Fig. 3). While the levels of soluble tau were reduced in cells treated with HTRA1 that were not seeded with fibril fragments, in agreement with previously published data (Tennstaedt et al., 2012), there were no marked HTRA1-dependent differences of soluble tau levels in cells treated with seeds.

### Example 4: Heterooligomer formation upon mixing of HTRA1 variants

To explore the possibility that internalised HTRA1 variants could form heteroligomers with native HTRA1 by the mechanism of monomer exchange, we incubated purified HTRA1 with equal amounts of a purified HTRA1 variant lacking its C-terminal PDZ domain (HTRA1ΔPDZ). Subsequently, theses samples were analysed by native mass spectrometry, a method allowing mass determination of protein complexes in solution (Fig. 4). These data reveal the presence of mixed trimers containing either HTRA1 with one or two PDZ domains, respectively, alongside with homotrimers of either HTRA1 and HTRA1ΔPDZ.

### Example 5: Uptake to HTRA1 into various mammalian cells

To test whether the observed uptake of exogenous HTRA1 is generally applicable, we tested additional cells for their ability to internalise HTRA1. We show that in addition to HEK293T cells (Fig. 1), SHSY5Y (Fig. 5 a,b), MEF (mouse embryonal fibrobalsts), SW480 and HeLa cells (Fig. 6) are also taking up exogenously applied HTRA1.

### Example 6: Uptake of cargo coupled to HTRA1

An obvious method to internalise cargo proteins into mammalian cells is to replace the PDZ domain of HTRA1 by other proteins (the cargo). To study whether HTRA1 can mediate uptake of cargo proteins, we constructed a plasmid producing a HTRA1ΔPDZ-GFP hybrid protein in *E. coli.* This protein was purified (Fig. 7a) and various amounts were applied to SW480 cells. Subsequently, Western blots of whole cell extracts were performed to detect internalization (Fig. 7b). These data indicate a dose dependent uptake similar to those observed with HTRA1 alone (compare Fig. 7b and Fig. 1a).

Moreover, as GFP is conveniently detectable because of its autofluorescence, we followed uptake of the HTRA1ΔPDZ-GFP hybrid protein in HeLa cells by confocal microscopy (Fig. 7c). These data indicate cytosolic localisation of the hybrid protein. In addition, free GFP migrated to the nucleus, suggesting that if the cargo is released from HTRA1, it can migrate or can be transported to a different cellular localisation.

### Example 7: Materials and methods

### (a) Internalisation of recombinant HTRA1 by 293T HEK cells

To study the spontaneous internalisation of recombinant HTRA1 protein from the medium, 8 x 10⁵ 293T HEK cells were seeded in poly-D-lysine coated 6 cm cell culture dishes and grown for 24 h to reach 50-60% confluency. Cells were washed twice with PBS before addition of 4 ml serum-free medium containing recombinant, labelled HTRA1 in the concentration and incubation time indicated. Subsequently, cells were detached from the culture dish by trypsin / EDTA treatment, centrifuged, and the cell pellet was washed thoroughly with PBS to remove all residual trypsin from the sample. The resulting pellet was lysed with RIPA buffer containing protease inhibitor (Roche). 150 µg total cell lysate were analysed by SDS PAGE followed by immunoblotting. Alternatively, 1.5 x 10⁵ cells were seeded in each well of a 24 well plate on poly-D-lysine coated glass coverslips, treated with 500 µl serum-free DMEM medium per well containing 50 µg/ml Alexa Fluor 568 labelled HTRA1 S328A, grown for 24 h, washed, methanol fixed and stained for immunofluorescence as described above. As a control for the uptake of labelled HTRA1, the amine reactive dye by itself was saturated by 1:5 dilution with 100 mM Tris, pH 8.42, and the same concentration as assessed by absorption at λ = 578 nm of the labelled protein and isolated dye was used.

Data obtained with other mammalian cells i.e. SW480, HeLa and SHSY5Y cells indicate that this method can be applied to any cultured mammalian cells.

### (b) Seeding of tau aggregation in 293T HEK cells

The seeding of intracellular tau aggregation in 293T HEK cells was done as described in the article text with some modifications and the additional analysis of the effect of HTRA1 internalised from the extracellular space.

*Preparation of MTBD aggregate seeds.* Fibril seeds composed of MTBD tau were prepared by fibrillisation for 24 h as described above, followed by ultracentrifugation at 186,000 x g, 4 °C for 1 h. The pellets were thoroughly resuspended with PBS, pH 7.4, vortexed, and the extent of fibrillization was checked by SDS-PAGE of samples from the supernatant and pellet. Typically, all of the MTBD tau was found in the pellet after 24 h of fibrillization. MTBD tau aggregates were sonicated in a water bath for 2 x 2 min before performing protein transfection.

*Transient transfection of 293T HEK cells and seeding of aggregation.* For sarkosyl extraction experiments, 293T HEK cells were transiently transfected by nucleofection (Lonza, Switzerland) with a pcDNA3.1 plasmid for overexpression of HA-tagged full-length human tau with the point mutation P301L. 4 x 10⁵ cells were grown in each well of a 6-well plate for expression of P301L tau for 24 h to reach about 50% confluency. Cell culture dishes were poly-L-lysine coated prior to seeding. Freshly prepared MTBD tau aggregate seeds were transfected into the cells at a final concentration of 17.5 µg/ml using 10 µl per well of the cationic lipid based protein transfection reagent Pro-Ject, followed by 4 h incubation with DMEM culture medium without serum and 20 h of incubation with 0.5% fetal bovine serum (FBS). For HTRA1 internalisation experiments, cells were washed twice with serum-free DMEM medium, followed by addition of 2 ml of medium conditioned with either PBS or HTRA1 at a final concentration of 150 µg/ml, i.e. 5.54 µM, and incubated at 37 °C for 20 h before performing sarkosyl extraction and immunoblotting.

### (c) Sarkosyl extraction of tau protein from cultured cells.

After seeded aggregation of tau proteins, sarkosyl extraction was performed to assess the abundance of aggregated versus soluble, HA-tagged P301L tau. Sarkosyl extraction was done essentially as described before (Guo and Lee, 2011) with some modifications. Cells were detached from the culture dish by trypsinisation and washed thoroughly with PBS to remove all residual trypsin from the cell pellet. The pellet was resuspended with lysis buffer containing sarkosyl, 10 mM Tris/HCl, 150 mM NaCl, 1 mM EGTA, 5 mM EDTA, 1% sarkosyl, pH 7.4 with protease inhibitor (Roche complete protease inhibitor tablet), and incubated on ice for 15 min. For cell lysis, the suspension was 10 x syringe sheared with a 27G needle, followed by incubation on ice for 15 min, 2 x 2 min sonication in the water bath and incubation at 25 °C for 20 min. The samples were ultracentrifuged at 186,000 x g, 4 °C for 60 min, the supernatant was saved as sarkosyl soluble fraction, the pellet was resuspended with the corresponding amount of SDS loading buffer containing 1% SDS by vigorous vortexing. Equal amounts of the individual samples were subjected to SDS-PAGE and immunoblotting based on the sarkosyl supernatant concentrations determined by absorption at 280 nm using a NanoDrop micro volume spectrophotometer (Peqlab, Germany). Volumes of the sarkosyl insoluble fractions were adjusted accordingly. The samples were loaded onto 10% SDS gels followed by immunoblotting against tau, HTRA1 and actin. The bands were detected by alkaline phosphatase (AP) coupled secondary antibodies, followed by chromogenic detection of AP activity.

### (d) Immunofluorescence and confocal laser-scanning microscopy

For seeding of tau aggregation and HTRA1 internalisation experiments to be analysed by confocal laser-scanning microscopy, the cells were transfected as done before for sarkosyl extraction except some modifications. Following transfection with tau P301L expression plasmids, 1.6 x 10⁵ cells were transferred to each well of 24 well plates with poly-D-lysine coated glass coverslips and grown for 24 h before transfection with MTBD seeds which were prepared as described above. For transfection in 24 well plates, 2.5 µl of the transfection reagent were used per well. Where indicated, treatment with labelled HTRA1 was done as described above except that 1.85 µM labelled HTRA1 was used in a final volume of 500 µl. After an incubation period of 20 h as described above, cells were fixed with ice-cold methanol, permeabilised with 0.5% Triton X-100 for 5 min, and washed with PBS before further staining was performed. For the detection of amyloid aggregates, Thioflavin S (ThS) staining was performed as described in the article text, by incubation with 0.005% ThS, dissolved in PBS and sterile filtered, for 8 min at RT, followed by 5 x washing with 50% ethanol for 5 min. The samples were then blocked with 5% bovine serum albumin (BSA) for 30 min before antibody staining was done with primary antibodies as indicated, using Alexa Fluor 633 labelled secondary antibodies. The anti HA antibody and secondary antibodies were diluted 1:500, the polyclonal anti HTRA1 PDZ antibody was used in 1:50 dilution in 3% BSA/PBS. Before mounting the samples with ProLong Gold antifade mounting solution (Life Technologies), they were washed with PBS 3 x for 15 min at RT. DAPI was added in 1:10,000 dilution together with the secondary antibody solutions. The nuclear counterstain To-Pro 3 iodide (Life Technologies) was used diluted 1:500 from a 1 mM stock solution. When To-Pro 3 staining was done, the samples were treated with 100 µg/ml RNAse A at 37 °C for 20 min to eliminate unspecific staining of cytoplasmic RNA. Microscopy was done with a Leica TCS SP5 Confocal Laser Scanning Microscope equipped with Leica HyD Gallium Arsenide phosphide hybrid detection systems. Image acquisition was performed with the same detector sensitivity settings for samples and controls. Images of the different channels were acquired individually in serial acquisition mode to avoid fluorescent bleed-through. Images of single focal planes using 60 µm pinhole width are shown.

### (e) Mixed trimer formation between HTRA1 and HTRA 1ΔPDZ

HTRA1 and HTRA1ΔPDZ (HTRA1 lacking its PDZ domain) were purified as described (Truebestein et al., 2011) except that a hydroxyapatite column (Bio-Rad) was added after the Ni-NTA purification step. Purified protein was concentrated to about 10 mg/ml in 100 mM NaH₂PO₄ buffer, pH= 8.

10 µM HTRA1 was mixed with 10 µM HTRA1ΔPDZ and incubated in 100 mM NaH₂PO₄ buffer, pH=8, for 5 min at 0°C or 37°C. Subsequently, the masses of proteins were analysed on a nanoESI-TOF mass spectrometer.

### (f) Uptake and cellular localization of HTRA1ΔPDZ-GFP into mammalian cells

*Cloning of the HTRA1*Δ*PDZ-GFP construct.* The inactive (S328A) protease domain of HTRA1 (HTRA1ΔPDZ) was amplified via PCR and cloned into a pET21d(+) vector (Novagen) followed by a six amino acid linker (EFGSGS) and full-length EGFP as well as a six amino acid His-tag for affinity purification.

*Purification of HTRA1*Δ*PDZ-GFP.* HTRA1ΔPDZ-GFP was expressed in Rosetta 2™ (DE3) cells (Invitrogen). Cells were lysed in lysis-buffer (20 mM Phosphate pH 7.4, 150 mM NaCl) using a Microfluidizer and cell debris was pelleted (35,000 g, 30 min). The cleared lysate was purified via affinity-purification (Protino NiTED Macherey & Nagel, 1 ml column volume) including several washing steps with lysis-buffer (3x) and high salt buffer (1 M NaCl, 1x). Proteins were eluted with 250 mM Imidazole (4x). Eluted fractions were pooled, concentrated and purified further via size exclusion chromatography in lysis-buffer (SEC, Superdex 200 10/300 column GE Healthcare).

*HTRA1*Δ*PDZ-GFP uptake.* SW480 cells were maintained in Roswell Park Memorial Institute 1640 Medium (RPMI 1640, Gibco) supplemented with 10% fetal bovine serum, 1% streptomycin and 1% penicillin at 37°C and 5% CO₂. 8x10⁵ SW480 cells were spilt into small tissue culture dishes (6 cm in diameter) in normal RPMI medium. After 24 h, cells were washed twice with serum free RPMI medium The purified HTRA1ΔPDZ-GFP protein was diluted in serum free medium to adjust concentrations to 0, 5, 20, 50, 100 or 150 µg protein per ml cell culture medium. PBS diluted in RPMI medium served as a control. A total volume of 2 ml was added to each dish. After 24 h, cells were washed in PBS, trypsinised for 15 min at 37°C and collected in serum free RPMI medium for sedimentation (5 min at 244 g). Cells were washed in PBS and sedimented. Pellets were resuspended in 100 µl lysis-buffer (50mM Tris HCl pH 7.4, 150 mM NaCl, 1% NP40, 0.5% Na-deoxycholate, 0.1% SDS, 1 mM EDTA) supplemented with 4 µl protease inhibitor (Complete protease inhibitor, Roche, 25x) and incubated on ice for 30 min. After sedimentation of cell debris (16,000 g, 15 min, 4°C) the supernatant was concentrated (Vivaspin columns, 10 kDa MWCO, Vivascience) and used for SDS-PAGE followed by Western bloting. Proteins were detected using an anti-GFP antibody (Abcam, ab5450, 1:1000) followed by an anti-goat-HRP labelled secondary antibody (Abcam, ab6741, 1:10,000) and ECL detection (SuperSignal West Pico Chemiluminescent Substrate, Thermo Scientific, 30 sec. exposure). Actin served as an internal loading control (anti-Actin antibody, MP Biomedicals 691001, 1:10,000 and anti-mouse-AP labelled secondary antibody, Sigma Aldrich 1418, 1:20,000).

*HTRA1protSA-GFP uptake and immunofluorescence anylsis.* HeLa cells were maintained in Dulbecco's Modified Eagle Medium GlutaMAX™ (DMEM, Gibco) supplemented with 1 % streptomycin, 1 % penicillin with or without 10 % fetal bovine serum (FBS), at 37 °C and 5 % CO2.

1x10⁵ HeLa cells were spilt into each well of a 12-well plate in DMEM medium with FBS. After 24 h, cells were washed twice with serum free DMEM medium. The purified HTRA1ΔPDZSA-GFP protein was diluted in serum free medium to a final concentration of 50 µg protein per ml medium. A total volume of 1 ml was added to each well for six h. Subsequently, cells were washed in PBS, trypsinised for 10 min at 37°C, collected in DMEM medium with FBS and plated into new wells with a coverslip (12 mm in diameter). After 24 h, cells were washed three times with PBS. Subsequently, cells were fixed with 4 % Paraformaldehyde (Sigma) diluted in PBS for 10 min at room temperature (RT) followed by washing twice with PBS. To permeabilise cells, they were incubated in PBS containing 0.5 % TritonX100 (Merck) for 5 min at RT, washed twice in PBS and then blocked with PBS containing 5 % Bovine Serum Albumin Fraction V (BSA, Roth) for 30 min. When the blocking solution was removed, PBS containing 2 % BSA and 0.2 % TritonX100 was supplemented with DAPI (Lifetechnologies, D3571, 1:10,000) and Alexa Fluor® 555 Phalloidin (Lifetechnologies, A34055, 1:500) and added to the samples for 45 min at RT in the dark. Cells were washed with PBS again four times, once with water, dried briefly and mounted onto glass slides with ProLong® Gold Antifade Mountant (Lifetechnologies). Samples were analysed at the Leica TCS SP5 microscope.

### REFERENCES

Bechara, C., and Sagan, S. (2013). Cell-penetrating peptides: 20 years later, where do we stand? FEBS Lett 587, 1693-1702.
Campioni, M., Severino, A., Manente, L., Tuduce, I.L., Toldo, S., Caraglia, M., Crispi, S., Ehrmann, M., He, X., Maguire, J., et al. (2010). The Serine Protease HtrA1 Specifically Interacts and Degrades the Tuberous Sclerosis Complex 2 Protein. Mol Cancer Res 8, 1248-1260.
Chien, J., Ota, T., Aletti, G., Shridhar, R., Boccellino, M., Quagliuolo, L., Baldi, A., and Shridhar, V. (2009). Serine protease HtrA1 associates with microtubules and inhibits cell migration. Mol Cell Biol 29, 4177-4187.
Clausen, T., Kaiser, M., Huber, R., and Ehrmann, M. (2011). HTRA proteases: regulated proteolysis in protein quality control. Nat Rev Mol Cell Biol 12, 152-162.
Clawson, G.A., Bui, V., Xin, P., Wang, N., and Pan, W. (2008). Intracellular localization of the tumor suppressor HtrA1/Prss11 and its association with HPV16 E6 and E7 proteins. J Cell Biochem 105, 81-88.
Eigenbrot, C., Ultsch, M., Lipari, M.T., Moran, P., Lin, S.J., Ganesan, R., Quan, C., Tom, J., Sandoval, W., van Lookeren Campagne, M., et al. (2012). Structural and functional analysis of HtrA1 and its subdomains. Structure 20, 1040-1050.
Guo, J.L., and Lee, V.M. (2011). Seeding of normal Tau by pathological Tau conformers drives pathogenesis of Alzheimer-like tangles. J Biol Chem 286, 15317-15331.
Hamidi, M., Zarrin, A., and Foroozesh, M. (2007). Novel delivery systems for interferons. Crit Rev Biotechnol 27, 111-127.
Shete, H.K., Prabhu, R.H., and Patravale, V.B. (2014). Endosomal escape: a bottleneck in intracellular delivery. J Nanosci Nanotechnol 14, 460-474.
Spillantini, M.G., and Goedert, M. (2013). Tau pathology and neurodegeneration. Lancet Neurol 12, 609-622.
Tennstaedt, A., Popsel, S., Truebestein, L., Hauske, P., Brockmann, A., Schmidt, N., Irle, I., Sacca, B., Niemeyer, C.M., Brandt, R., et al. (2012). Human High Temperature Requirement Serine Protease A1 (HTRA1) Degrades Tau Protein Aggregates. J Biol Chem 287, 20931-20941.
Ter-Avetisyan, G., Tunnemann, G., Nowak, D., Nitschke, M., Herrmann, A., Drab, M., and Cardoso, M.C. (2009). Cell entry of arginine-rich peptides is independent of endocytosis. J Biol Chem 284, 3370-3378.
Truebestein, L., Tennstaedt, A., Monig, T., Krojer, T., Canellas, F., Kaiser, M., Clausen, T., and Ehrmann, M. (2011). Substrate-induced remodeling of the active site regulates human HTRA1 activity. Nat Struct Mol Biol 18, 386-388.

## Claims

1. An in vitro method for modulating the HTRA1 activity inside a cell, comprising the steps of
(a) contacting the cell with a HTRA1 polypeptide in the absence of any other means suitable for inducing uptake of polypeptides into a cell;
(b) incubating the cell in the presence of the HTRA1 polypeptide for a period of time sufficient for uptake of the HTRA1 polypeptide into the cell;
wherein the cell is a mammalian cell; and
wherein the HTRA1 polypeptide comprises the HTRA1 protease domain or an amino acid sequence derived therefrom which shares an identity with the amino acid sequence of the HTRA1 protease domain over its entire length of at least 80%.

2. The method according to claim 1 for increasing the HTRA1 activity in the cell, wherein the HTRA1 polypeptide
(i) has protease activity; and/or
(ii) comprises the amino acid sequence selected from the group consisting of amino acid positions 158 to 373 of SEQ ID NO: 1, amino acid positions 158 to 480 of SEQ ID NO: 1, or amino acid positions 23 to 480 of SEQ ID NO: 1, or an amino acid sequence which shares an identity with any one of these amino acid sequences over their entire length of at least 80%; and/or
(iii) is a constitutively active HTRA1 variant.

3. The method according to claim 1 for decreasing the HTRA1 activity in the cell, wherein the HTRA1 polypeptide
(i) does not have any protease activity, wherein preferably the amino acid residue corresponding to serine at position 328 of SEQ ID NO: 1 is any amino acid residue except serine, such as alanine; and/or
(ii) is a dominant negative HTRA1 variant; and/or
(iii) is a dominant negative HTRA1 variant which has a reduced ability to form trimers.

4. The method according to any one of claims 1 to 3, wherein the cell is contacted with a HTRA1 polypeptide in a concentration of at least 1 µg/ml, preferably at least 10 µg/ml or at least 50 µg/ml; and/or wherein the cell is incubated in the presence of the HTRA1 polypeptide for at least 1 min, preferably at least 10 min.

5. A HTRA1 polypeptide for use in the treatment of a disease in a human being which benefits from increasing the HTRA1 activity, wherein a pharmaceutical preparation containing the HTRA1 polypeptide is locally applied to the site of the disease in order to modulate the HTRA1 activity inside the affected cells, wherein the pharmaceutical preparation does not contain any other means suitable for inducing uptake of polypeptides into a cell, wherein the HTRA1 polypeptide has protease activity and comprises the HTRA1 protease domain or an amino acid sequence derived therefrom which shares an identity with the amino acid sequence of the HTRA1 protease domain over its entire length of at least 80%; and wherein
(i) the disease is a tauopathy such as Alzheimer's disease and the HTRA1 polypeptide is locally applied to the brain; or
(ii) the disease is cancer comprising cancer cells having a decreased HTRA1 activity and the HTRA1 polypeptide is locally applied to the tumor site.

6. An in vitro method for transport of a molecule of interest into a cell, comprising the steps of
(a) providing a conjugate of the molecule of interest and a HTRA1 polypeptide;
(b) contacting the cell with the conjugate;
(c) incubating the cell in the presence of the conjugate for a period of time sufficient for uptake of the conjugate into the cell;
wherein the HTRA1 polypeptide comprises the HTRA1 protease domain or an amino acid sequence derived therefrom which shares an identity with the amino acid sequence of the HTRA1 protease domain over its entire length of at least 80%.

7. The method according to claim 6, wherein the molecule of interest is a polypeptide.

8. The method according to claim 7, wherein the molecule of interest and the HTRA1 polypeptide
(i) are covalently conjugated to each other; and/or
(ii) are fused together and form a hybrid polypeptide; and/or
(iii) are conjugated via a disulfide bond; and/or
(iv) are non-covalently bound to each other, wherein the HTRA1 polypeptide preferably comprises a PDZ domain and the molecule of interest comprises a PDZ binding peptide.

9. The method according to any one of claims 6 to 8, wherein the HTRA1 polypeptide
(i) does not have any protease activity, wherein preferably the amino acid residue corresponding to serine at position 328 of SEQ ID NO: 1 is any amino acid residue except serine, such as alanine; and/or
(ii) is mutated so as to reduce its substrate binding affinity.

10. The method according to any one of claims 6 to 9, wherein the cell is contacted with the conjugate in a concentration of at least 1 µg/ml, preferably at least 10 µg/ml or at least 50 µg/ml; and/or wherein the cell is incubated in the presence of the conjugate for at least 1 min, preferably at least 10 min.

11. A conjugate comprising a therapeutic agent and a HTRA1 polypeptide, wherein the HTRA1 polypeptide comprises the HTRA1 protease domain or an amino acid sequence derived therefrom which shares an identity with the amino acid sequence of the HTRA1 protease domain over its entire length of at least 80%.

12. The conjugate according to claim 11 for use in the treatment of a disease, wherein a pharmaceutical preparation containing the conjugate is locally applied to the site of the disease.

## Patentansprüche

1. In-vitro-Verfahren zur Modulation der HTRA1 Aktivität im Inneren einer Zelle, umfassend die folgenden Schritte
(a) Inkontaktbringen der Zelle mit einem HTRA1 Polypeptid in Abwesenheit eines anderen Mittels, das geeignet ist, die Aufnahme von Polypeptiden in eine Zelle zu induzieren;
(b) Inkubation der Zelle in Gegenwart des HTRA1 Polypeptids über einen Zeitraum, der für die Aufnahme des HTRA1 Polypeptids in die Zelle ausreicht;
wobei die Zelle eine Säugetierzelle ist; und
wobei das HTRA1 Polypeptid die HTRA1 Proteasedomäne oder eine davon abgeleitete Aminosäuresequenz umfasst, die eine Identität von mindestens 80% mit der Aminosäuresequenz der HTRA1 Proteasedomäne über ihre gesamte Länge teilt.

2. Das Verfahren nach Anspruch 1 zur Erhöhung der HTRA1 Aktivität in der Zelle, wobei das HTRA1 Polypeptid
(i) Proteaseaktivität besitzt; und/oder
(ii) eine Aminosäuresequenz umfasst, ausgewählt aus der Gruppe bestehend aus den Aminosäurepositionen 158 bis 373 der SEQ ID NO: 1, den Aminosäurepositionen 158 bis 480 der SEQ ID NO: 1 oder den Aminosäurepositionen 23 bis 480 der SEQ ID NO: 1, oder einer Aminosäuresequenz, die eine Identität von mindestens 80% mit einer dieser Aminosäuresequenzen über ihre gesamte Länge teilt; und/oder
(iii) eine konstitutiv aktive HTRA1 Variante ist.

3. Das Verfahren nach Anspruch 1 zur Reduktion der HTRA1 Aktivität in der Zelle, wobei das HTRA1 Polypeptid
(i) keine Proteaseaktivität aufweist, wobei vorzugsweise der Aminosäurerest, der dem Serin an Position 328 von SEQ ID NO: 1 entspricht, ein beliebiger Aminosäurerest außer Serin, wie beispielsweise Alanin, ist; und/oder
(ii) eine dominante negative HTRA1 Variante ist; und/oder
(iii) eine dominante negative HTRA1 Variante ist, die eine verringerte Fähigkeit zur Bildung von Trimeren aufweist.

4. Das Verfahren nach einem der Ansprüche 1 bis 3, wobei die Zelle mit einem HTRA1 Polypeptid in einer Konzentration von mindestens 1 µg/ml, vorzugsweise mindestens 10 µg/ml oder mindestens 50 µg/ml, in Kontakt gebracht wird; und/oder wobei die Zelle in Gegenwart des HTRA1 Polypeptids für mindestens 1 min, vorzugsweise mindestens 10 min, inkubiert wird.

5. HTRA1 Polypeptid zur Verwendung bei der Behandlung einer Krankheit bei einem Menschen, die von einer Erhöhung der HTRA1 Aktivität profitiert, wobei eine pharmazeutische Zubereitung, die das HTRA1 Polypeptid enthält, lokal an der Stelle der Krankheit appliziert wird, um die HTRA1 Aktivität innerhalb der betroffenen Zellen zu modulieren, wobei die pharmazeutische Zubereitung kein anderes Mittel enthält, das geeignet ist, die Aufnahme von Polypeptiden in eine Zelle zu induzieren, wobei das HTRA1 Polypeptid Proteaseaktivität aufweist und die HTRA1 Proteasedomäne oder eine davon abgeleitete Aminosäuresequenz umfasst, die eine Identität von mindestens 80% mit der Aminosäuresequenz der HTRA1 Proteasedomäne über ihre gesamte Länge teilt;
und wobei
(i) die Krankheit eine Tauopathie wie die Alzheimer-Krankheit ist und das HTRA1 Polypeptid lokal im Gehirn appliziert wird; oder
(ii) die Krankheit Krebs, umfassend Krebszellen mit einer verminderten HTRA1 Aktivität, ist und das HTRA1 Polypeptid lokal an der Tumorstelle appliziert wird.

6. In-vitro-Verfahren zum Transport eines Moleküls von Interesse in eine Zelle, umfassend die Schritte
(a) Bereitstellung eines Konjugats des Moleküls von Interesse und einem HTRA1 Polypeptid;
(b) Inkontaktbringen der Zelle mit dem Konjugat;
(c) Inkubieren der Zelle in Gegenwart des Konjugats für einen Zeitraum, der ausreichend für die Aufnahme des Konjugats in die Zelle ist;
wobei das HTRA1 Polypeptid die HTRA1 Proteasedomäne oder eine davon abgeleitete Aminosäuresequenz umfasst, die eine Identität von mindestens 80% mit der Aminosäuresequenz der HTRA1 Proteasedomäne über ihre gesamte Länge teilt.

7. Das Verfahren nach Anspruch 6, wobei das Molekül von Interesse ein Polypeptid ist.

8. Das Verfahren nach Anspruch 7, wobei das Molekül von Interesse und das HTRA1 Polypeptid
(i) kovalent aneinander konjugiert sind; und/oder
(ii) aneinander fusioniert sind und ein hybrides Polypeptid bilden; und/oder
(iii) über eine Disulfidbindung konjugiert sind; und/oder
(iv) nicht kovalent aneinander gebunden sind, wobei das HTRA1 Polypeptid vorzugsweise eine PDZ Domäne umfasst und das Molekül von Interesse ein PDZ-bindendes Peptid umfasst.

9. Das Verfahren nach einem der Ansprüche 6 bis 8, wobei das HTRA1 Polypeptid
(i) keine Proteaseaktivität aufweist, wobei vorzugsweise der Aminosäurerest, der dem Serin an Position 328 der SEQ ID NO: 1 entspricht, ein beliebiger Aminosäurerest außer Serin, wie beispielsweise Alanin, ist; und/oder
(ii) so mutiert ist, dass seine Substratbindungsaffinität reduziert ist.

10. Das Verfahren nach einem der Ansprüche 6 bis 9, wobei die Zelle mit dem Konjugat in einer Konzentration von mindestens 1 µg/ml, vorzugsweise mindestens 10 µg/ml oder mindestens 50 µg/ml, in Kontakt gebracht wird; und/oder wobei die Zelle in Gegenwart des Konjugats für mindestens 1 min, vorzugsweise mindestens 10 min, inkubiert wird.

11. Konjugat umfassend ein Therapeutikum und ein HTRA1 Polypeptid, wobei das HTRA1 Polypeptid die HTRA1 Proteasedomäne oder eine davon abgeleitete Aminosäuresequenz umfasst, die eine Identität von mindestens 80% mit der Aminosäuresequenz der HTRA1 Proteasedomäne über ihre gesamte Länge teilt.

12. Das Konjugat nach Anspruch 11 zur Verwendung bei der Behandlung einer Krankheit, wobei eine pharmazeutische Zubereitung, die das Konjugat enthält, lokal an der Stelle der Krankheit appliziert wird.

## Revendications

1. Procédé in vitro destiné à moduler l'activité de HTRA1 à l'intérieur d'une cellule, comprenant les étapes consistant à :
(a) mettre en contact la cellule avec un polypeptide de HTRA1 en l'absence de tout autre moyen approprié pour induire l'introduction de polypeptides dans une cellule ;
(b) incuber la cellule en présence du polypeptide de HTRA1 sur un intervalle de temps suffisant pour l'introduction du polypeptide de HTRA1 dans la cellule ;
où la cellule est une cellule de mammifère ; et
où le polypeptide de HTRA1 comprend le domaine de protéase HTRA1 ou une séquence d'acides aminés dérivée de celui-ci laquelle partage une homologie avec la séquence d'acides aminés du domaine de protéase HTRA1 sur toute sa longueur d'au moins 80 %.

2. Procédé selon la revendication 1 destiné à augmenter l'activité de HTRA1 dans la cellule, où le polypeptide de HTRA1
(i) présente une activité de protéase ; et/ou
(ii) comprend la séquence d'acides aminés sélectionnée parmi le groupe constitué des positions d'acides aminés 158 à 373 de la séquence à numéro d'identification SEQ ID N° : 1, des positions d'acides aminés 158 à 480 de la séquence SEQ ID N° : 1, ou des positions d'acides aminés 23 à 480 de la séquence SEQ ID N° : 1, ou une séquence d'acides aminés qui partage une homologie avec l'une quelconque de ces séquences d'acides aminés sur toute leur longueur d'au moins 80 % ; et/ou
(iii) est un variant de HTRA1 constitutivement actif.

3. Procédé selon la revendication 1 destiné à diminuer l'activité de HTRA1 dans la cellule, où le polypeptide de HTRA1
(i) ne présente pas d'activité de protéase, où de préférence le résidu d'acide aminé correspondant à la sérine à la position 328 de la séquence SEQ ID N° : 1 est tout résidu d'acide aminé autre que la sérine, tel que l'alanine ; et/ou
(ii) est un variant de HTRA1 négatif dominant ; et/ou
(iii) est un variant de HTRA1 négatif dominant qui a une aptitude réduite à former des trimères.

4. Procédé selon l'une quelconque des revendications 1 à 3, dans lequel la cellule est mise en contact avec un polypeptide de HTRA1 à une concentration d'au moins 1 µg/ml, de préférence d'au moins 10 µg/ml ou d'au moins 50 µg/ml ; et/ou dans lequel la cellule est incubée en présence du polypeptide de HTRA1 pendant au moins 1 min, de préférence au moins 10 min.

5. Polypeptide de HTRA1 destiné à être utilisé dans le traitement d'une maladie chez l'être humain qui bénéficie d'une augmentation de l'activité de HTRA1, où une préparation pharmaceutique contenant le polypeptide de HTRA1 est appliquée localement au niveau du site de la maladie de sorte à moduler l'activité de HTRA1 à l'intérieur des cellules affectées, où la préparation pharmaceutique ne contient aucun autre moyen approprié pour induire l'introduction de polypeptides dans une cellule, où le polypeptide de HTRA1 présente une activité de protéase et comprend le domaine de protéase HTRA1 ou une séquence d'acides aminés dérivée de celui-ci laquelle partage une homologie avec la séquence d'acides aminés du domaine de protéase HTRA1 sur toute sa longueur d'au moins 80 % ; et où
(i) la maladie est une tauopathie telle que la maladie d'Alzheimer et le polypeptide est appliqué localement au niveau du cerveau ; ou
(ii) la maladie est un cancer comprenant des cellules cancéreuses ayant une activité de HTRA1 réduite et le polypeptide est appliqué localement au niveau du site tumoral.

6. Procédé in vitro de transport d'une molécule d'intérêt dans une cellule, comprenant les étapes consistant à
(a) fournir un conjugué de la molécule d'intérêt et d'un polypeptide de HTRA1 ;
(b) mettre en contact la cellule avec le conjugué ;
(c) incuber la cellule en présence du conjugué sur un intervalle de temps suffisant pour l'introduction du conjugué dans la cellule ;
où le polypeptide de HTRA1 comprend le domaine de protéase HTRA1 ou une séquence d'acides aminés dérivée de celui-ci laquelle partage une homologie avec la séquence d'acides aminés du domaine de protéase HTRA1 sur toute sa longueur d'au moins 80 %.

7. Procédé selon la revendication 6, dans lequel la molécule d'intérêt est un polypeptide.

8. Procédé selon la revendication 7, dans lequel la molécule d'intérêt et le polypeptide de HTRA1
(i) sont conjugués de manière covalente l'un à l'autre ; et/ou
(ii) sont fusionnés ensemble et forment un polypeptide hybride ; et/ou
(iii) sont conjugués par un pont disulfure ; et/ou
(iv) sont liés de manière non covalente l'un à l'autre, où le polypeptide de HTRA1 comprend de préférence un domaine PDZ et la molécule d'intérêt comprend un peptide de liaison de PDZ.

9. Procédé selon l'une quelconque des revendications 6 à 8, dans lequel le polypeptide de HTRA1
(i) ne présente pas d'activité de protéase, où de préférence le résidu d'acide aminé correspondant à la sérine à la position 328 de la séquence SEQ ID N° : 1 est tout résidu d'acide aminé autre que la sérine, tel que l'alanine ; et/ou
(ii) est muté de sorte à réduire son affinité de liaison au substrat.

10. Procédé selon l'une quelconque des revendications 6 à 9, dans lequel la cellule est mise en contact avec le conjugué à une concentration d'au moins 1 µg/ml, de préférence d'au moins 10 µg/ml ou d'au moins 50 µg/ml ; et/ou dans lequel la cellule est incubée en présence du conjugué pendant au moins 1 min, de préférence au moins 10 min.

11. Conjugué comprenant un agent thérapeutique et un polypeptide de HTRA1, où le polypeptide de HTRA1 comprend le domaine de protéase HTRA1 ou une séquence d'acides aminés dérivée de celui-ci laquelle partage une homologie avec la séquence d'acides aminés du domaine de protéase HTRA1 sur toute sa longueur d'au moins 80 %.

12. Conjugué selon la revendication 11, destiné à être utilisé dans le traitement d'une maladie, où une préparation pharmaceutique contenant le conjugué est appliquée localement au niveau du site de la maladie.


### SEQUENCE LISTING

<110> Universität Duisburg-Essen
<120> Internalisation of human HTRA1 and cargo proteins into mammalian cells
<130> 56 988 K
<150> EP15162558.9
   <151> 2015-04-07
<160> 16
<170> PatentIn version 3.5
<210> 1
   <211> 480
   <212> PRT
   <213> Homo sapiens
<400> 1
<210> 2
   <211> 480
   <212> **PRT**
   <213> Homo sapiens
<400> 2
   Met Gln Ile Pro Arg Ala Ala Leu Leu Pro Leu Leu Leu Leu Leu Leu
   1               5                   10                  15
   Ala Ala Pro Ala Ser Ala Gln Leu Ser Arg Ala Gly Arg Ser Ala Pro
               20                  25                  30
   Leu Ala Ala Gly Cys Pro Asp Arg Cys Glu Pro Ala Arg Cys Pro Pro
           35                  40                  45
   Gln Pro Glu His Cys Glu Gly Gly Arg Ala Arg Asp Ala Cys Gly Cys
       50                  55                  60
   Cys Glu Val Cys Gly Ala Pro Glu Gly Ala Ala Cys Gly Leu Gln Glu
   65                  70                  75                  80
   Gly Pro Cys Gly Glu Gly Leu Gln Cys Val Val Pro Phe Gly Val Pro
                   85                  90                  95
   Ala Ser Ala Thr Val Arg Arg Arg Ala Gln Ala Gly Leu Cys Val Cys
               100                 105                 110
   Ala Ser Ser Glu Pro Val Cys Gly Ser Asp Ala Asn Thr Tyr Ala Asn
           115                 120                 125
   Leu Cys Gln Leu Arg Ala Ala Ser Arg Arg Ser Glu Arg Leu His Arg
       130                 135                 140
   Pro Pro Val Ile Val Leu Gln Arg Gly Ala Cys Gly Gln Gly Gln Glu
   145                 150                 155                 160
   Asp Pro Asn Ser Leu Arg His Lys Tyr Asn Phe Ile Ala Asp Val Val
   165 170 175
   Glu Lys Ile Ala Pro Ala Val Val His Ile Glu Leu Phe Arg Lys Leu
   180 185 190
   Pro Phe Ser Lys Arg Glu Val Pro Val Ala Ser Gly Ser Gly Phe Ile
   195 200 205
   Val Ser Glu Asp Gly Leu Ile Val Thr Asn Ala His Val Val Thr Asn
   210 215 220
   Lys His Arg Val Lys Val Glu Leu Lys Asn Gly Ala Thr Tyr Glu Ala
   225 230 235 240
   Lys Ile Lys Asp Val Asp Glu Lys Ala Asp Ile Thr Leu Ile Lys Ile
   245 250 255
   Asp His Gln Gly Lys Leu Pro Val Leu Leu Leu Gly Arg Ser Ser Glu
   260 265 270
   Leu Arg Pro Gly Glu Phe Val Val Ala Ile Gly Ser Pro Phe Ser Leu
   275 280 285
   Gln Asn Thr Val Thr Thr Gly Ile Val Ser Thr Thr Gln Arg Gly Gly
   290 295 300
   Lys Glu Leu Gly Leu Arg Asn Ser Asp Met Asp Tyr Ile Gln Thr Asp
   305 310 315 320
   Ala Ile Ile Asn Tyr Gly Asn Ser Gly Gly Pro Leu Val Asn Leu Asp
   325 330 335
   Gly Glu Val Ile Gly Ile Asn Thr Leu Lys Val Thr Ala Gly Ile Ser
   340 345 350
   Phe Ala Ile Pro Ser Asp Lys Ile Lys Lys Phe Leu Thr Glu Ser His
   355 360 365
   Asp Arg Gln Ala Lys Gly Lys Ala Ile Thr Lys Lys Lys Tyr Ile Gly
   370 375 380
   Ile Arg Met Met Ser Leu Thr Ser Ser Lys Ala Lys Glu Leu Lys Asp
   385 390 395 400
   Arg His Arg Asp Phe Pro Asp Val Ile Ser Gly Ala Tyr Ile Ile Glu
   405 410 415
   Val Ile Pro Asp Thr Pro Ala Glu Ala Gly Gly Leu Lys Glu Asn Asp
   420 425 430
   Val Ile Ile Ser Ile Asn Gly Gln Ser Val Val Ser Ala Asn Asp Val
   435 440 445
   Ser Asp Val Ile Lys Arg Glu Ser Thr Leu Asn Met Val Val Arg Arg
   450 455 460
   Gly Asn Glu Asp Ile Met Ile Thr Val Ile Pro Glu Glu Ile Asp Pro
   465 470 475 480
<210> 3
   <211> 480
   <212> **PRT**
   <213> Homo sapiens
<400> 3
   Met Gln Ile Pro Arg Ala Ala Leu Leu Pro Leu Leu Leu Leu Leu Leu
   1 5 10 15
   Ala Ala Pro Ala Ser Ala Gln Leu Ser Arg Ala Gly Arg Ser Ala Pro
   20 25 30
   Leu Ala Ala Gly Cys Pro Asp Arg Cys Glu Pro Ala Arg Cys Pro Pro
   35 40 45
   Gln Pro Glu His Cys Glu Gly Gly Arg Ala Arg Asp Ala Cys Gly Cys
   50 55 60
   Cys Glu Val Cys Gly Ala Pro Glu Gly Ala Ala Cys Gly Leu Gln Glu
   65 70 75 80
   Gly Pro Cys Gly Glu Gly Leu Gln Cys Val Val Pro Phe Gly Val Pro
   85 90 95
   Ala Ser Ala Thr Val Arg Arg Arg Ala Gln Ala Gly Leu Cys Val Cys
   100 105 110
   Ala Ser Ser Glu Pro Val Cys Gly Ser Asp Ala Asn Thr Tyr Ala Asn
   115 120 125
   Leu Cys Gln Leu Arg Ala Ala Ser Arg Arg Ser Glu Arg Leu His Arg
   130 135 140
   Pro Pro Val Ile Val Leu Gln Arg Gly Ala Cys Gly Gln Gly Gln Glu
   145 150 155 160
   Asp Pro Asn Ser Leu Arg His Lys Tyr Asn Phe Ile Ala Asp Val Val
                   165                 170                 175
   Glu Lys Ile Ala Pro Ala Val Val His Ile Glu Leu Phe Arg Lys Leu
               180                 185                 190
   Pro Phe Ser Lys Arg Glu Val Pro Val Ala Ser Gly Ser Gly Phe Ile
           195                 200                 205
   Val Ser Glu Asp Gly Leu Ile Val Thr Asn Ala His Val Val Thr Asn
       210                 215                 220
   Lys His Arg Val Lys Val Glu Leu Lys Asn Gly Ala Thr Tyr Glu Ala
   225                 230                 235                 240
   Lys Ile Lys Asp Val Asp Glu Lys Ala Asp Ile Ala Leu Ile Lys Ile
                   245                 250                 255
   Asp His Gln Gly Lys Leu Pro Val Leu Leu Leu Gly Arg Ser Ser Glu
               260                 265                 270
   Leu Arg Pro Gly Glu Phe Val Val Ala Ile Gly Ser Pro Phe Ser Leu
           275                 280                 285
   Gln Asn Thr Val Thr Thr Gly Ile Met Ser Thr Thr Gln Arg Gly Gly
       290                 295                 300
   Lys Glu Leu Gly Leu Arg Asn Ser Asp Met Asp Tyr Ile Gln Thr Asp
   305                 310                 315                 320
   Ala Ile Ile Asn Tyr Gly Asn Ser Gly Gly Pro Leu Val Asn Leu Asp
                   325                 330                 335
   Gly Glu Val Ile Gly Ile Asn Thr Leu Lys Val Thr Ala Gly Ile Ser
               340                 345                 350
   Phe Ala Ile Pro Ser Asp Lys Ile Lys Lys Phe Leu Thr Glu Ser His
           355                 360                 365
   Asp Arg Gln Ala Lys Gly Lys Ala Ile Thr Lys Lys Lys Tyr Ile Gly
       370                 375                 380
   Ile Arg Met Met Ser Leu Thr Ser Ser Lys Ala Lys Glu Leu Lys Asp
   385                 390                 395                 400
   Arg His Arg Asp Phe Pro Asp Val Ile Ser Gly Ala Tyr Ile Ile Glu
                   405                 410                 415
   Val Ile Pro Asp Thr Pro Ala Glu Ala Gly Gly Leu Lys Glu Asn Asp
               420                 425                 430
   Val Ile Ile Ser Ile Asn Gly Gln Ser Val Val Ser Ala Asn Asp Val
           435                 440                 445
   Ser Asp Val Ile Lys Arg Glu Ser Thr Leu Asn Met Val Val Arg Arg
       450                 455                 460
   Gly Asn Glu Asp Ile Met Ile Thr Val Ile Pro Glu Glu Ile Asp Pro
   465                 470                 475                 480
<210> 4
   <211> 453
   <212> **PRT**
   <213> Homo sapiens
<400> 4
   Met Gln Ala Arg Ala Leu Leu Leu Ala Ala Leu Ala Ala Leu Ala Leu
   1               5                   10                  15
   Ala Arg Glu Pro Pro Ala Ala Pro Cys Pro Ala Arg Cys Asp Val Ser
               20                  25                  30
   Arg Cys Pro Ser Pro Arg Cys Pro Gly Gly Tyr Val Pro Asp Leu Cys
           35                  40                  45
   Asn Cys Cys Leu Val Cys Ala Ala Ser Glu Gly Glu Pro Cys Gly Gly
       50                  55                  60
   Pro Leu Asp Ser Pro Cys Gly Glu Ser Leu Glu Cys Val Arg Gly Leu
   65                  70                  75                  80
   Cys Arg Cys Arg Trp Ser His Ala Val Cys Gly Thr Asp Gly His Thr
                   85                  90                  95
   Tyr Ala Asn Val Cys Ala Leu Gln Ala Ala Ser Arg Arg Ala Leu Gln
               100                 105                 110
   Leu Ser Gly Thr Pro Val Arg Gln Leu Gln Lys Gly Ala Cys Pro Leu
           115                 120                 125
   Gly Leu His Gln Leu Ser Ser Pro Arg Tyr Lys Phe Asn Phe Ile Ala
       130                 135                 140
   Asp Val Val Glu Lys Ile Ala Pro Ala Val Val His Ile Glu Leu Phe
   145 150 155 160
   Leu Arg His Pro Leu Phe Gly Arg Asn Val Pro Leu Ser Ser Gly Ser
   165 170 175
   Gly Phe Ile Met Ser Glu Ala Gly Leu Ile Ile Thr Asn Ala His Val
   180 185 190
   Val Ser Ser Asn Ser Ala Ala Pro Gly Arg Gln Gln Leu Lys Val Gln
   195 200 205
   Leu Gln Asn Gly Asp Ser Tyr Glu Ala Thr Ile Lys Asp Ile Asp Lys
   210 215 220
   Lys Ser Asp Ile Ala Thr Ile Lys Ile His Pro Lys Lys Lys Leu Pro
   225 230 235 240
   Val Leu Leu Leu Gly His Ser Ala Asp Leu Arg Pro Gly Glu Phe Val
   245 250 255
   Val Ala Ile Gly Ser Pro Phe Ala Leu Gln Asn Thr Val Thr Thr Gly
   260 265 270
   Ile Val Ser Thr Ala Gln Arg Glu Gly Arg Glu Leu Gly Leu Arg Asp
   275 280 285
   Ser Asp Met Asp Tyr Ile Gln Thr Asp Ala Ile Ile Asn Tyr Gly Asn
   290 295 300
   Ser Gly Gly Pro Leu Val Asn Leu Asp Gly Glu Val Ile Gly Ile Asn
   305 310 315 320
   Thr Leu Lys Val Thr Ala Gly Ile Ser Phe Ala Ile Pro Ser Asp Arg
   325 330 335
   Ile Thr Arg Phe Leu Thr Glu Phe Gln Asp Lys Gln Ile Lys Asp Trp
   340 345 350
   Lys Lys Arg Phe Ile Gly Ile Arg Met Arg Thr Ile Thr Pro Ser Leu
   355 360 365
   Val Asp Glu Leu Lys Ala Ser Asn Pro Asp Phe Pro Glu Val Ser Ser
   370 375 380
   Gly Ile Tyr Val Gln Glu Val Ala Pro Asn Ser Pro Ser Gln Arg Gly
   385 390 395 400
   Gly Ile Gln Asp Gly Asp Ile Ile Val Lys Val Asn Gly Arg Pro Leu
   405 410 415
   Val Asp Ser Ser Glu Leu Gln Glu Ala Val Leu Thr Glu Ser Pro Leu
   420 425 430
   Leu Leu Glu Val Arg Arg Gly Asn Asp Asp Leu Leu Phe Ser Ile Ala
   435 440 445
   Pro Glu Val Val Met
   450
<210> 5
   <211> 476
   <212> **PRT**
   <213> Homo sapiens
<400> 5
   Met Ile Arg Pro Gln Leu Arg Thr Ala Gly Leu Gly Arg Cys Leu Leu
   1 5 10 15
   Pro Gly Leu Leu Leu Leu Leu Val Pro Val Leu Trp Ala Gly Ala Glu
   20 25 30
   Lys Leu His Thr Gln Pro Ser Cys Pro Ala Val Cys Gln Pro Thr Arg
   35 40 45
   Cys Pro Ala Leu Pro Thr Cys Ala Leu Gly Thr Thr Pro Val Phe Asp
   50 55 60
   Leu Cys Arg Cys Cys Arg Val Cys Pro Ala Ala Glu Arg Glu Val Cys
   65 70 75 80
   Gly Gly Ala Gln Gly Gln Pro Cys Ala Pro Gly Leu Gln Cys Leu Gln
   85 90 95
   Pro Leu Arg Pro Gly Phe Pro Ser Thr Cys Gly Cys Pro Thr Leu Gly
   100 105 110
   Gly Ala Val Cys Gly Ser Asp Arg Arg Thr Tyr Pro Ser Met Cys Ala
   115 120 125
   Leu Arg Ala Glu Asn Arg Ala Ala Arg Arg Leu Gly Lys Val Pro Ala
   130 135 140
   Val Pro Val Gln Trp Gly Asn Cys Gly Asp Thr Gly Thr Arg Ser Ala
   145 150 155 160
   Gly Pro Leu Arg Arg Asn Tyr Asn Phe Ile Ala Ala Val Val Glu Lys
                   165                 170                 175
   Val Ala Pro Ser Val Val His Val Gln Leu Trp Gly Arg Leu Leu His
               180                 185                 190
   Gly Ser Arg Leu Val Pro Val Tyr Ser Gly Ser Gly Phe Ile Val Ser
           195                 200                 205
   Glu Asp Gly Leu Ile Ile Thr Asn Ala His Val Val Arg Asn Gln Gln
       210                 215                 220
   Trp Ile Glu Val Val Leu Gln Asn Gly Ala Arg Tyr Glu Ala Val Val
   225                 230                 235                 240
   Lys Asp Ile Asp Leu Lys Leu Asp Leu Ala Val Ile Lys Ile Glu Ser
                   245                 250                 255
   Asn Ala Glu Leu Pro Val Leu Met Leu Gly Arg Ser Ser Asp Leu Arg
               260                 265                 270
   Ala Gly Glu Phe Val Val Ala Leu Gly Ser Pro Phe Ser Leu Gln Asn
           275                 280                 285
   Thr Ala Thr Ala Gly Ile Val Ser Thr Lys Gln Arg Gly Gly Lys Glu
       290                 295                 300
   Leu Gly Met Lys Asp Ser Asp Met Asp Tyr Val Gln Ile Asp Ala Thr
   305                 310                 315                 320
   Ile Asn Tyr Gly Asn Ser Gly Gly Pro Leu Val Asn Leu Asp Gly Asp
                   325                 330                 335
   Val Ile Gly Val Asn Ser Leu Arg Val Thr Asp Gly Ile Ser Phe Ala
               340                 345                 350
   Ile Pro Ser Asp Arg Val Arg Gln Phe Leu Ala Glu Tyr His Glu His
           355                 360                 365
   Gln Met Lys Gly Lys Ala Phe Ser Asn Lys Lys Tyr Leu Gly Leu Gln
       370                 375                 380
   Met Leu Ser Leu Thr Val Pro Leu Ser Glu Glu Leu Lys Met His Tyr
   385                 390                 395                 400
   Pro Asp Phe Pro Asp Val Ser Ser Gly Val Tyr Val Cys Lys Val Val
                   405                 410                 415
   Glu Gly Thr Ala Ala Gln Ser Ser Gly Leu Arg Asp His Asp Val Ile
               420                     425                     430
   Val Asn Ile Asn Gly Lys Pro Ile Thr Thr Thr Thr Asp Val Val Lys
           435                     440                     445
   Ala Leu Asp Ser Asp Ser Leu Ser Met Ala Val Leu Arg Gly Lys Asp
       450                     455                     460
   Asn Leu Leu Leu Thr Val Ile Pro Glu Thr Ile Asn
   465                 470                     475
<210> 6
   <211> 7
   <212> PRT
   <213> Artificial Sequence
<220>
   <223> HTRA1 peptide loop
<220>
   <221> MISC_FEATURE
   <222> (7)..(7)
   <223> Xaa is Pro or Glu
<400> 6
   Asp Pro Met Phe Lys Leu Xaa
   1               5
<210> 7
   <211> 8
   <212> **PRT**
   <213> Artificial Sequence
<220>
   <223> cellular localization signal
<400> 7
   Pro Pro Lys Lys Lys Arg Leu Val
   1               5
<210> 8
   <211> 9
   <212> PRT
   <213> Artificial Sequence
<220>
   <223> cellular localization signal
<220>
   <221> MISC_FEATURE
   <222> (3)..(8)
   <223> Xaa may be any amino acid
<400> 8
   Arg Leu Xaa Xaa Xaa Xaa Xaa His Leu
   1               5
<210> 9
   <211> 25
   <212> PRT
   <213> Artificial Sequence
<220>
   <223> cellular localization signal
<400> 9
   Met Leu Ser Leu Arg Gln Ser Ile Arg Phe Phe Lys Pro Ala Thr Arg
   1               5                   10                  15
   Thr Leu Cys Ser Ser Arg Tyr Leu Leu
               20                  25
<210> 10
   <211> 4
   <212> PRT
   <213> Artificial Sequence
<220>
   <223> PDZ binding peptide
<220>
   <221> MISC_FEATURE
   <222> (1)..(1)
   <223> Xaa is Leu, Val or Ile
<220>
   <221> MISC_FEATURE
   <222> (2)..(2)
   <223> Xaa is Thr or Leu
<220>
   <221> MISC_FEATURE
   <222> (3)..(3)
   <223> Xaa is Leu, Phe, Tyr or Thr
<220>
   <221> MISC_FEATURE
   <222> (4)..(4)
   <223> Xaa is Leu, Val, Ala or Ile
<400> 10
   Xaa Xaa Xaa Xaa
   1
<210> 11
   <211> 4
   <212> PRT
   <213> Artificial Sequence
<220>
   <223> PDZ binding peptide
<220>
   <221> MISC_FEATURE
   <222> (1)..(1)
   <223> Xaa is a hydrophobic or non-polar amino acid
<220>
   <221> MISC_FEATURE
   <222> (2)..(2)
   <223> Xaa is any amino acid
<220>
   <221> MISC_FEATURE
   <222> (3)..(3)
   <223> Xaa is a hydrophobic or non-polar amino acid
<220>
   <221> MISC_FEATURE
   <222> (4)..(4)
   <223> Xaa is Val, Leu, Phe or Ala
<400> 11
   Xaa Xaa Xaa Xaa
   1
<210> 12
   <211> 4
   <212> PRT
   <213> Artificial Sequence
<220>
   <223> PDZ binding peptide
<220>
   <221> MISC_FEATURE
   <222> (1)..(1)
   <223> Xaa is Thr, Ile or Cys
<220>
   <221> MISC_FEATURE
   <222> (2)..(2)
   <223> Xaa is Trp or Phe
<220>
   <221> MISC_FEATURE
   <222> (3)..(3)
   <223> Xaa is Leu, Ile, Phe or Trp
<220>
   <221> MISC_FEATURE
   <222> (4)..(4)
   <223> Xaa is Leu or Val
<400> 12
   Xaa Xaa Xaa Xaa
   1
<210> 13
   <211> 11
   <212> PRT
   <213> Artificial Sequence
<220>
   <223> PDZ binding peptide
<400> 13
   Leu Phe Lys Trp Leu Gln Leu Thr Met Phe Ala
   1 5 10
<210> 14
   <211> 11
   <212> PRT
   <213> Artificial Sequence
<220>
   <223> PDZ binding peptide
<400> **14**
   Asp Leu Ile Ser Trp Leu Cys Phe Ser Val Leu
   1 5 10
<210> 15
   <211> 11
   <212> PRT
   <213> Artificial Sequence
<220>
   <223> PDZ binding peptide
<400> 15
   Met Asp Gln Leu Ala Phe His Gln Phe Tyr Ile
   1 5 10
<210> 16
   <211> 11
   <212> PRT
   <213> Artificial Sequence
<220>
   <223> PDZ binding peptide
<400> 16
   Ser Tyr Ala Ala Trp Ile Asp Ser Val Leu Ala
   1 5 10